# EUROPÄISCHE PATENTSCHRIFT

(11) **EP 1 007 602 B1**
(45) Veröffentlichungstag und Bekanntmachung des Hinweises auf die Patenterteilung: **07.12.2005**
(21) Anmeldenummer: 98943857.7
(22) Anmeldetag: 06.08.1998
(51) Int. Cl.: C09K 9/02, G02F 1/15

(54) **UV-GESCHÜTZTE ELEKTROCHROME LÖSUNG**
UV-PROTECTED ELECTROCHROMIC SOLUTION
SOLUTION ELECTROCHROME PROTEGEE CONTRE LES UV

(30) Priorität: 18.08.1997 DE 19735732
(43) Veröffentlichungstag der Anmeldung: 14.06.2000
(73) Patentinhaber: Bayer Innovation GmbH, 40225 Düsseldorf (DE)
(72) Erfinder: BERNETH, Horst, D-51373 Leverkusen (DE); HEUER, Helmut-Werner, D-47829 Krefeld (DE); NEIGL, Ralf, D-51373 Leverkusen (DE)
(86) Internationale Anmeldenummer: PCT/EP1998/004910
(87) Internationale Veröffentlichungsnummer: WO 1999/009112

(56) Entgegenhaltungen:
- EP-A- 0 430 684
- EP-A- 0 431 547
- EP-A- 0 491 364
- EP-A- 0 612 826
- WO-A-97/30134
- WO-A-98/05736
- US-A- 5 280 380
- YONEMURA ET AL: "Anomalously stable cyclodextrin complexes of phenothiazine-viologen linked compounds with a long spacer chain" TETRAHEDRON LETTERS, Bd. 30, Nr. 24, 1989, Seiten 3143-3146, XP002085157
- PANETTA ET AL: "TTF-NHCO2(CH2)2O-TCNQBr and TTF-CO2(CH2)2O-TCNQBr, two potential molecular rectifiers" MOLECULAR CRYSTALS AND LIQUID CRYSTALS, Bd. 107, 1984, Seiten 103-113, XP002085159
- BAUER ET AL: "Inter- und intramolekularer Energietransfer und Ladungstrennung in kovalent gebundenen Cyanin-Farbstoff-Viologen-Systemen" ZEITSCHRIFT FÜR NATURFORSHUNG, Bd. 48b, 1993, Seiten 461-471, XP002085161
- SIMONSEN, KLAUS B. ET AL: "Stable Macrocyclic and Tethered Donor Acceptor Systems. Intramolecular Bipyridinium and Tetrathiafulvalene Assemblies" J. ORG. CHEM. (1997), 62(3), 679-686 CODEN: JOCEAH;ISSN: 0022-3263, XP002085162
- SCHMELZER ET AL: "New LB heterostructures containing electron donating and accepting molecules" MOLECULAR CRYSTALS AND LIQUID CRYSTALS SCIENCE AND TECHNOLOGY SECTION A, Bd. 253, 1994, Seiten 173-181, XP002085366

## Beschreibung

Die vorliegende Erfindung betrifft eine UV-geschützte elektrochrome Lösung, deren Verwendung in einer elektrochromen Vorrichtung sowie eine elektrochrome Vorrichtung, die diese Lösung enthält.

Elektrochrome Vorrichtungen, die ein elektrochromes System enthalten, sind bereits bekannt.

Solche Vorrichtungen enthalten als elektrochromes System üblicherweise Paare von Redoxsubstanzen, die in einem inerten Lösungsmittel gelöst sind. Zusätzlich können Leitsalze, Lichtstabilisatoren und Substanzen, die die Viskosität beeinflussen, enthalten sein.

Als Paar von Redoxsubstanzen wird je eine reduzierbare und eine oxidierbare Substanz verwendet. Beide sind farblos oder nur schwach gefärbt. Unter Einfluss einer elektrischen Spannung wird die eine Substanz reduziert, die andere oxidiert, wobei wenigstens eine farbig wird. Nach Abschalten der Spannung bilden sich die beiden ursprünglichen Redoxsubstanzen wieder zurück, wobei Entfärbung bzw. Farbaufhellung auftritt.

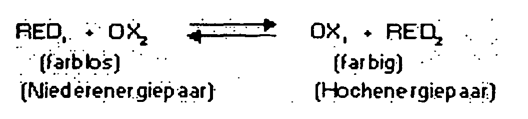

Aus US-4.902.108 ist bekannt, dass solche Paare von Redoxsubstanzen geeignet sind, bei denen die reduzierbare Substanz wenigstens zwei chemisch reversible Reduktionswellen im cyclischen Voltammogramm und die oxidierbare Substanz entsprechend wenigstens zwei chemisch reversible Oxidationswellen besitzt.

Elektrochrome Vorrichtungen können auf vielfältige Weise Anwendung finden. So können sie z.B. als Automobilrückspiegel ausgebildet sein, der bei Nachtfahrt durch Anlegen einer Spannung abgedunkelt werden kann und somit das Blenden durch Scheinwerfer fremder Fahrzeuge verhindert wird (vgl. z.B. US-3.280.701, US-4.902.108, EP-A-0.435.689). Weiterhin können solche Vorrichtungen auch in Fensterscheiben oder Autosonnendächern eingesetzt werden, wo sie nach Anlegen einer Spannung das Sonnenlicht abdunkeln. Letztlich kann mit solchen Vorrichtungen auch eine Anzeigevorrichtung aufgebaut werden zur bildlichen Darstellung von Informationen wie Buchstaben, Zahlen und Zeichen.

Elektrochrome Vorrichtungen bestehen normalerweise aus einem Paar Glas- oder Kunststoffscheiben, von denen im Falle eines Autospiegels eine verspiegelt ist. Eine Seite dieser Scheiben ist mit einer lichtdurchlässigen, elektrisch leitfähigen Schicht, z.B. Indium-Zinn-Oxid (ITO), beschichtet. Aus diesen Scheiben wird nun eine Zelle aufgebaut, indem sie mit ihrer einander zugewandten elektrisch leitfähig beschichteten Seite mit einem ringförmigen oder rechteckigen Dichtungsring verbunden, vorzugsweise verklebt werden. Der Dichtungsring stellt einen gleichmäßigen Abstand zwischen den Scheiben her, beispielsweise 0,1 bis 0,5 mm. In diese Zelle wird nun über eine Öffnung eine elektrochrome Lösung eingefüllt und die Zelle dicht verschlossen. Über die ITO-Schicht lassen sich die beiden Scheiben getrennt kontaktieren.

Bei den aus dem Stand der Technik bekannten elektrochromen Systemen sind solche Paare von Redoxsubstanzen enthalten, die nach Reduktion bzw. Oxidation farbige Radikale, Kationradikale oder Anionradikale bilden, die chemisch reaktiv sind. Wie beispielsweise aus Topics in Current Chemistry, Vol. 92, S. 1-44 (1980) bekannt ist, können solche Radikal(ionen) empfindlich gegenüber Elektrophilen oder Nukleophilen oder auch Radikalen sein. Es muss deshalb zum Erreichen einer hohen Stabilität einer elektrochromen Vorrichtung, die ein solches elektrochromes System enthält, das mehrere tausend Schaltcyclen überstehen soll, dafür gesorgt werden, dass das verwendete Lösungsmittel absolut frei von Elektrophilen, z.B. Protonen, Nukleophilen und Sauerstoff ist. Weiterhin muss dafür gesorgt werden, dass sich solche reaktiven Spezies nicht durch elektrochemische Prozesse an den Elektroden während des Betriebs der elektrochromen Vorrichtung bilden..

Die gemäß obiger Reaktionsgleichung formulierte Rückreaktion zu RED₁ und OX₂ erfolgt auch während des Betriebs der elektrochromen Vorrichtung kontinuierlich abseits der Elektroden innerhalb des Lösungsvolumens. Wegen der beschriebenen Gefahren von Abbaureaktionen der Radikal-(ionen) durch Elektrophile, Nukleophile oder Radikale ist es für die Langzeitstabilität des Displays wichtig, dass die Rückreaktion gemäß obiger Reaktionsgleichung möglichst rasch und ohne Nebenreaktionen erfolgen kann.

Solche elektrochromen Vorrichtungen zeigen in der Regel eine Empfindlichkeit gegenüber Licht, insbesondere UV-Licht. Es sind deshalb auch elektrochrome Vorrichtungen bekannt, die UV-Stabilisatoren enthalten, beispielsweise aus US-5.280.380.

Es wurde nun gefunden, dass durch Kopplung von RED₁ und OX₂ über eine kovalente chemische Bindung die Elektronenübertragung erleichtert wird und somit die Rückreaktion gemäß obiger Reaktionsgleichung beschleunigt und Nebenreaktionen vermieden werden können.

Es wurde ebenfalls gefunden, dass elektrochrome Lösungen, die solche über eine kovalente chemische Bindung verbrückte RED₁ und OX₂ enthalten, durch besondere UV-Absorber effektiv gegen Zerstörung durch UV-Licht geschützt werden können.

Die vorliegende Erfindung betrifft demnach eine elektrochrome Lösung, enthaltend mindestens eine oxidierbare Substanz RED₁, die durch Elektronenabgabe an einer Anode, und mindestens eine reduzierbare Substanz OX₂, die durch Elektronenaufnahme an einer Kathode, unter Zunahme der Extinktion im sichtbaren Bereich des Spektrums von einer schwach gefärbten oder farblosen Form in eine gefärbte Form OX₁ bzw. RED₂ übergeht, wobei nach Ladungsausgleich die schwach gefärbte bzw. farblose Form zurückgebildet wird, dadurch gekennzeichnet,
dass sie mindestens eine elektrochrome Substanz der Formeln

OX₂-B-RED₁ (Ia),

OX₂-B-RED₁-B-OX₂ (Ib),

RED₁-B-OX₂-B-RED₁ (Ic),

oder

OX₂-(B-RED₁-B-OX₂)_{d}-B-RED₁ (Id),

worin
- d: für eine ganze Zahl von 1 bis 5 steht,
- OX₂: für einen Rest der Formeln 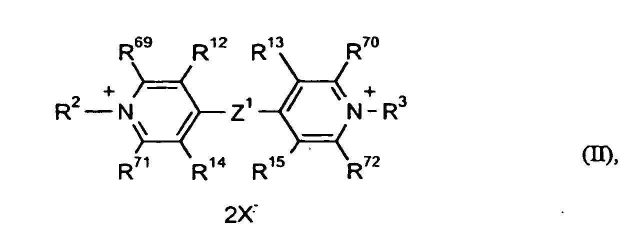 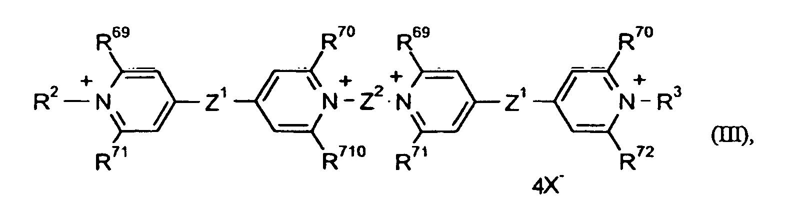 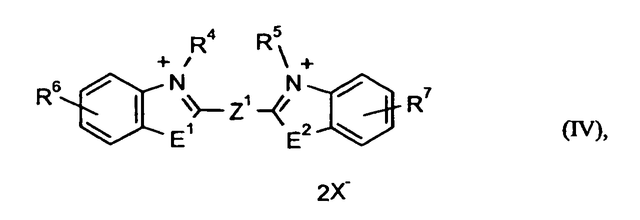 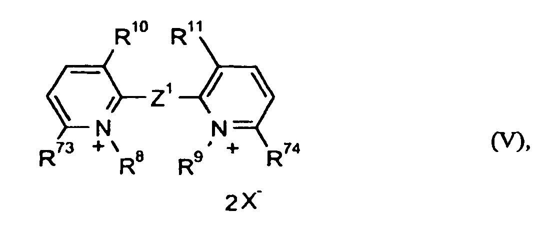 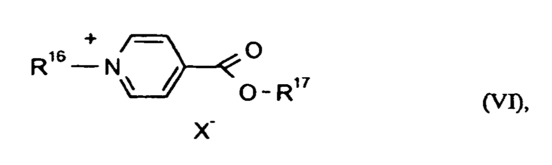 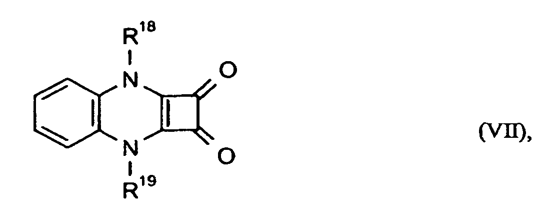 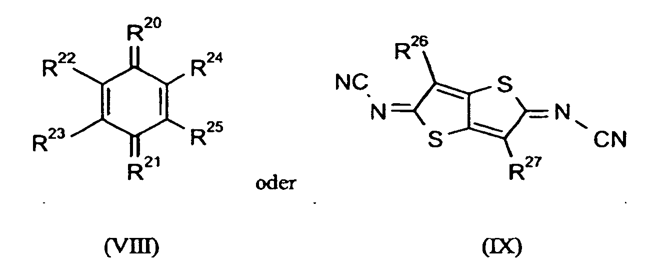
steht, wobei
- R² bis R⁵, R⁸, R⁹, R¹⁶ bis R¹⁹: unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten oder
- R⁴ und R⁵ oder R⁸ und R⁹: gemeinsam eine -(CH₂)₂- oder -(CH₂)₃-Brücke bilden,
- R⁶, R⁷ und R²² bis R²⁵: unabhängig voneinander Wasserstoff, C₁- bis C₄-Alkyl, C₁- bis C₄-Alkoxy, Halogen, Cyano, Nitro oder C₁- bis C₄-Alkoxycarbonyl bedeuten oder
- R²² und R²³ und/oder R²⁴ und R²⁵: eine -CH=CH-CH=CH-Brücke bilden,
- R¹⁰ und R¹¹; R¹² und R¹³; R¹⁴ und R¹⁵: unabhängig voneinander Wasserstoff oder paarweise eine -(CH₂)₂-, -(CH₂)₃- oder -CH=CH-Brucke bedeuten,
- R⁶⁹ bis R⁷⁴: unabhängig voneinander Wasserstoff oder C₁-C₆-Alkyl bedeuten, oder
- R⁶⁹; R¹² und/oder R⁷⁰; R¹³: eine -CH=CH-CH=CH-Brücke bilden,
- R²⁰: und R²¹ unabhängig voneinander O, N-CN, C(CN)₂ oder N-C₆- bis C₁₀-Aryl bedeuten,
- R²⁶: Wasserstoff, C₁- bis C₄-Alkyl, C₁- bis C₄-Alkoxy, Halogen, Cyano, Nitro, C₁- bis C₄-Alkoxy-carbonyl oder C₆- bis C₁₀-Aryl bedeutet,
- E¹ und E²: unabhängig voneinander O, S, NR¹ oder C(CH₃)₂ bedeuten oder
- E¹ und E²: gemeinsam eine -N-(CH₂)₂-N-Brücke bilden,
- R¹: C₁- bis C₁₈-Alkyl, C₂- bis C₁₂-Alkenyl, C₄- bis C₇-Cycloalkyl, C₇- bis C₁₅-Aralkyl, C₆- bis C₁₀-Aryl bedeutet,
- Z¹: eine direkte Bindung, -CH=CH-, -C(CH₃)=CH-, -C(CN)=CH-, -CCl=CCl-, -C(OH)=CH-, -CCl=CH-, -C≡C-, -CH=N-N=CH-, -C(CH₃)=N-N=C(CH₃)- oder -CCl=N-N=CCl- bedeutet,
- Z²: -(CH₂)ᵣ- oder -CH₂-C₆H₄-CH₂- bedeutet,
- r: eine ganze Zahl von 1 bis 10 bedeutet und
- X⁻: ein unter den Bedingungen redox-inertes Anion bedeutet,
wobei die Bindung zum Brückenglied B über einen der Reste R²-R¹⁹, R²²-R²⁷ oder im Falle, dass E¹ oder E² für NR¹ steht über R¹ erfolgt und die genannten Reste dann für eine direkte Bindung stehen,
- RED₁: für einen der folgenden Reste 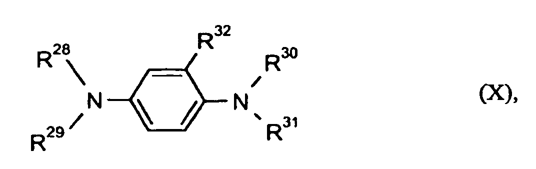 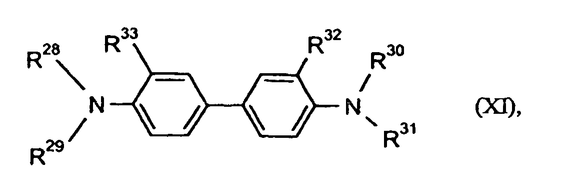 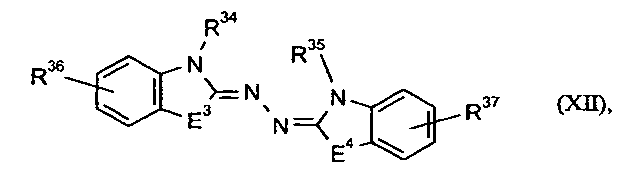 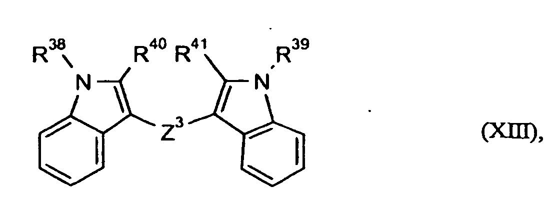 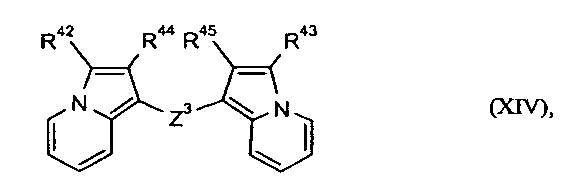 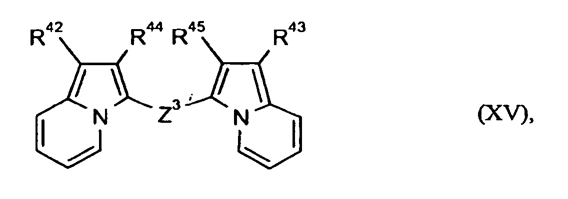 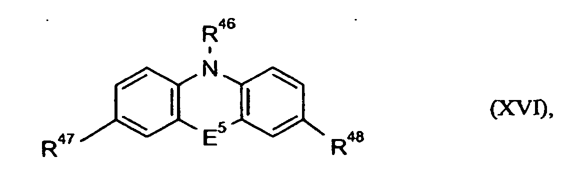 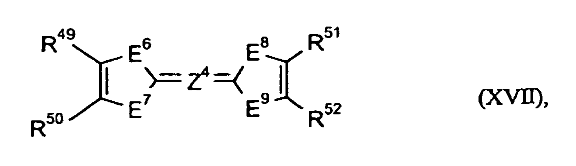 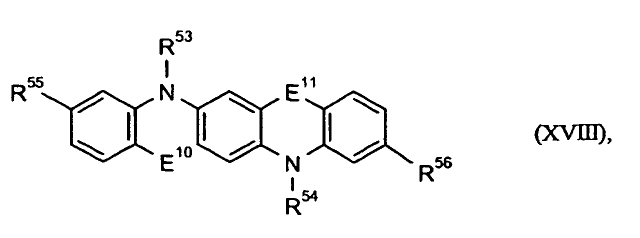 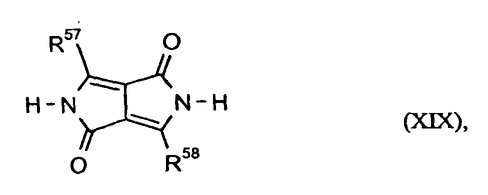 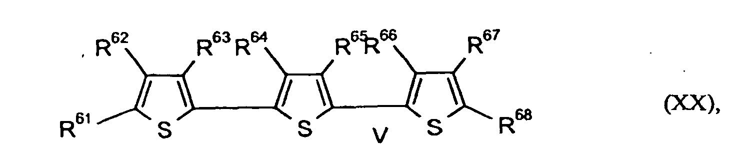
steht, worin
- R²⁸ bis R³¹, R³⁴, R³⁵, R³⁸, R³⁹, R⁴⁶, R⁵³ und R⁵⁴: unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten, und R⁴⁶, R⁵³ und R⁵⁴ zusätzlich Wasserstoff bedeuten,
- R³², R³³, R³⁶, R³⁷, R⁴⁰, R⁴¹, R⁴² bis R⁴⁵, R⁴⁷, R⁴⁸, R⁴⁹ bis R⁵² und R⁵⁵ bis R⁵⁷: unabhängig voneinander Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeuten und R⁵⁷ und R⁵⁸ zusätzlich einen gegebenenfalls benzanellierten aromatischen oder quasiaromatischen fünf- oder sechsgliedrigen heterocyclischen Ring bedeuten und R⁴⁸ zusätzlich NR⁷⁵R⁷⁶ bedeutet,
- R⁴⁹ und R⁵⁰: und/oder R⁵¹ und R⁵² eine -(CH₂)₃-, -(CH₂)₄-, -(CH₂)₅- oder -CH=CH-CH=CH-Brücke bilden,
- Z³: eine direkte Bindung, eine -CH=CH- oder -N=N-Brücke bedeutet,
- =Z⁴=: eine direkte Doppelbindung, eine =CH-CH= oder =N-N=-Brücke bedeutet,
- E³ bis E⁵, E¹⁰ und E¹¹: unabhängig voneinander O, S, NR⁵⁹ oder C(CH₃)₂ bedeuten und E⁵ zusätzlich C = O oder SO₂ bedeutet, oder
- E³ und E⁴: unabhängig voneinander -CH=CH- bedeuten,
- E⁶ bis E⁹: unabhängig voneinander S, Se oder NR⁵⁹ bedeuten,
- R⁵⁹, R⁷⁵ und R⁷⁶: unabhängig voneinander C₁- bis C₁₂-Alkyl, C₂- bis C₈-Alkenyl, C₃- bis C₇-Cycloalkyl, C₇- bis C₁₅-Aralkyl oder C₆- bis C₁₀-Aryl bedeuten und R⁷⁵ zusätzlich Wasserstoff bedeutet oder
- R⁷⁵ und R⁷⁶: in der Bedeutung von NR⁷⁵R⁷⁶ gemeinsam mit dem N-Atom, an das sie gebunden sind, einen fünf- oder sechsgliedrigen, gesättigten Ring bilden, der weitere Heteroatome enthalten kann,
- R⁶¹ bis R⁶⁸: unabhängig voneinander Wasserstoff, C₁- bis C₆-Alkyl, C₁- bis C₄-Alkoxy, Cyano, C₁- bis C₄-Alkoxycarbonyl oder C₆- bis C₁₀-Aryl bedeuten, oder
- R⁶¹; R⁶² und R⁶⁷; R⁶⁸: unabhängig voneinander gemeinsam eine -(CH₂)₃-, -(CH₂)₄- oder -CH=CH-CH=CH-Brücke bilden,
- v: eine ganze Zahl zwischen 0 und 10 bedeutet,
wobei die Bindung zum Brückenglied B über einen der Reste R²⁸-R⁵⁸, R⁶¹, R⁶², R⁶⁷, R⁶⁸ oder im Falle, dass einer der Reste E³-E¹¹ für NR⁵⁹ steht über R⁵⁹ erfolgt und die genannten Reste dann für eine direkte Bindung stehen, und
- B: für ein Brückenglied der Formeln -(CH₂)ₙ- oder -[Y¹ₛ(CH₂)ₘ-Y²]ₒ-(CH₂)ₚ-Y³_{q}- steht, welche jeweils gegebenenfalls durch C₁- bis C₄-Alkoxy, Halogen oder Phenyl substituiert sind,
- Y¹ bis Y³: unabhängig voneinander für O, S, NR⁶⁰, COO, CONH, NHCONH, Cyclopentandiyl, Cyclohexandiyl, Phenylen oder Naphthylen stehen,
- R⁶⁰: C₁- bis C₆-Alkyl, C₂- bis C₆-Alkenyl, C₄- bis C₇-Cycloalkyl, C₇- bis C₁₅-Aralkyl oder C₆- bis C₁₀-Aryl bedeutet,
- n: eine ganze Zahl von 1 bis 12 bedeutet,
- m und p: unabhängig voneinander eine ganze Zahl von 0 bis 8 bedeuten,
- o: eine ganze Zahl von 0 bis 6 bedeutet und
- q und s: unabhängig voneinander 0 oder 1 bedeuten,
und mindestens einen UV-Absorber der Formel 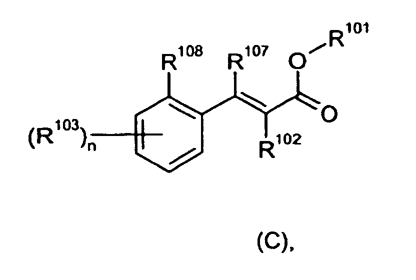 enthält,
worin
- R¹⁰¹: für gegebenenfalls verzweigtes C₁- bis C₂₀-Alkyl steht,
- R¹⁰²: für Wasserstoff, Cyano oder COOR¹ steht,
- R¹⁰³: für Wasserstoff, C₁- bis C₁₂-Alkyl oder C₁- bis C₁₂-Alkoxy steht,
- R¹⁰⁷: gemeinsam mit R¹⁰⁸ eine C₂- oder C₃-Brücke bilden, die bis zu 3 C₁- bis C₄-Alkylreste tragen kann, und
- n: für eine ganze Zahl von 1 bis 3 steht.

Die Reduktions- und Oxidationsprozesse in dem erfindungsgemäßen elektrochromen System erfolgen im Allgemeinen durch Elektronenaufnahme bzw. -abgabe an einer Kathode bzw. Anode, wobei zwischen den Elektroden vorzugsweise eine Potentialdifferenz von 0,3 bis 3 V herrscht. Nach Abschalten des elektrischen Potentials erfolgt im Allgemeinen spontan ein Ladungsausgleich zwischen den Substanzen RED₂ und OX₁, wobei eine Entfärbung bzw. Farbaufhellung eintritt. Ein solcher Ladungsausgleich erfolgt auch bereits während des Stromflusses im Innern des Elektrolytvolumens.

Ganz besonders bevorzugt ist eine erfindungsgemäße elektrochrome Lösung, welche mindestens eine Substanz der Formel (Ia)-(Id) enthält,
worin
- OX₂: für einen Rest der Formeln (II), (III), (IV) oder (V) steht,
wobei
- R², R³, R⁴, R⁵, R⁸ und R⁹: unabhängig voneinander für C₁- bis C₁₂-Alkyl, C₂- bis C₈-Alkenyl, C₅- bis C₇-Cycloalkyl, C₇-bis C₁₅-Aralkyl oder C₆- bis C₁₀-Aryl stehen,
- R⁶ und R⁷: unabhängig voneinander für Wasserstoff, Methyl, Ethyl, Methoxy, Ethoxy, Fluor, Chlor, Brom, Cyano, Nitro, Methoxycarbonyl oder Ethoxycarbonyl stehen,
- R¹⁰, R¹¹; R¹², R¹³ und R¹⁴, R¹⁵: unabhängig voneinander für Wasserstoff oder, falls Z¹ eine direkte Bindung bedeutet, jeweils gemeinsam für eine -(CH₂)₂-, -(CH₂)₃- oder -CH=CH-Brücke stehen,
oder
- R⁴, R⁵ und R⁸, R⁹: unabhängig voneinander paarweise gemeinsam für eine -(CH₂)₂- oder -(CH₂)₃-Brücke stehen, falls Z¹ eine direkte Bindung bedeutet,
- R⁶⁹ bis R⁷⁴: unabhängig voneinander Wasserstoff oder C₁-C₄-Alkyl bedeuten,
- E¹ und E²: gleich sind und für O, S, NR¹ oder C(CH₃)₂ stehen oder gemeinsam eine -N-(CH₂)₂-N-Brücke bilden,
- R¹: für C₁- bis C₁₂-Alkyl, C₂- bis C₄-Alkenyl, C₅- bis C₇-Cycloalkyl, C₇- bis C₁₅-Aralkyl oder C₆-bis C₁₀-Aryl steht,
- Z¹: für eine direkte Bindung, -CH=CH-, -C(CH₃)=CH-, -C(CN)=CH-, -C≡C- oder -CH=N-N=CH- steht,
- Z²: für -(CH)ᵣ- oder -CH₂-C₆H₄-CH₂- steht,
- r: für eine ganze Zahl zwischen 1 und 6 steht,
- X⁻: für ein unter den Bedingungen redox-inertes, farbloses Anion steht,
wobei die Bindung zum Brückenglied B über einen der Reste R²-R¹¹ oder im Falle, dass E¹ oder E² für NR¹ steht über R¹ erfolgt und die genannten Reste dann für eine direkte Bindung stehen,
- RED₁: für einen Rest der Formeln (X), (XI), (XII), (XIII), (XVI), (XVII), (XVIII) oder (XX) steht,
wobei
- R²⁸ bis R³¹, R³⁴, R³⁵, R³⁸, R³⁹, R⁴⁶, R⁵³ und R⁵⁴: unabhängig voneinander C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl bedeuten und
- R⁴⁶, R⁵³ und R⁵⁴: zusätzlich Wasserstoff bedeuten,
- R³², R³³, R³⁶, R³⁷, R⁴⁰, R⁴¹, R⁴⁷ bis R⁵², R⁵⁵ und R⁵⁶: unabhängig voneinander Wasserstoff, Methyl, Ethyl, Methoxy, Ethoxy, Fluor, Chlor, Brom, Cyan, Nitro, Methoxycarbonyl, Ethoxycarbonyl oder Phenyl bedeuten und
- R⁵⁷ und R⁵⁸: zusätzlich 2- oder 4-Pyridyl bedeuten und
- R⁴⁸: zusätzlich NR⁷⁵R⁷⁶ bedeutet,
- Z³: eine direkte Bindung, eine -CH=CH- oder -N=N-Brücke bedeutet,
- =Z⁴=: eine direkte Doppelbindung, eine =CH-CH= oder =N-N=Brücke bedeutet,
- E³ bis E⁵, E¹⁰ und E¹¹: unabhängig voneinander O, S, NR⁵⁹ oder C(CH₃)₂ bedeuten, E³ und E⁴ aber die gleiche Bedeutung haben,
- E⁶ bis E⁹: untereinander gleich sind und S, Se oder NR⁵⁹ bedeuten und
- E⁵: zusätzlich C = O bedeutet,
- E⁶: für NR⁵⁹ steht, wobei R⁵⁹ eine direkte Bindung zur Brücke B bedeutet und
- E⁷ bis E⁹: die oben angegebene Bedeutung besitzen, aber untereinander nicht gleich sein müssen,
- R⁵⁹, R⁷⁵ und R⁷⁶: unabhängig voneinander C₁- bis C₁₂-Alkyl, C₂- bis C₈-Alkenyl, C₅- bis C₇-Cycloalkyl, C₇- bis C₁₅-Aralkyl oder C₆- bis C₁₀-Aryl bedeuten, und R⁷⁵ zusätzlich Wasserstoff bedeutet oder
- R⁷⁵ und R⁷⁶: in der Bedeutung NR⁷⁵R⁷⁶ gemeinsam mit dem N-Atom, an das sie gebunden sind, Pyrrolidino, Piperidino oder Morpholino bedeuten,
- R⁶¹, R⁶² und R⁶⁷, R⁶⁸: unabhängig voneinander für Wasserstoff, C₁- bis C₄-Alkyl, Methoxycarbonyl, Ethoxycarbonyl oder Phenyl oder paarweise gemeinsam für eine -(CH₂)₃- oder -(CH₂) ₄-Brücke stehen,
- R⁶³ bis R⁶⁶: für Wasserstoff stehen und
- v: für eine ganze Zahl von 1 bis 6 steht,
wobei die Bindung zum Brückenglied B über einen der Reste R²⁸-R⁴¹, R⁴⁶-R⁵⁶, R⁶¹, R⁶², R⁶⁷, R⁶⁸ oder im Falle, dass einer der Reste E³-E¹¹ für NR⁵⁹ steht, über R⁵⁹ erfolgt und die genannten Reste dann für eine direkte Bindung stehen,
- B: für ein Brückenglied der Formeln -(CH₂)ₙ-, -(CH₂)ₘ-O-(CH₂)ₚ-, -(CH)ₘ-NR⁶⁰-(CH₂)ₚ-, -(CH₂) ₘ-C₆H₄-(CH₂)ₚ-, -[O-(CH₂)ₚ]ₒ-O-, -[NR⁶⁰-(CH₂)ₚ]ₒ-NR⁶⁰-, -[C₆H₄-(CH₂)ₚ]ₒ-C₆H₄-, -(CH₂) ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H ₄-NHCONH-(CH₂)ₚ-, -(CH₂)ₘ-OCO-(CH₂)ₜ-COO-(CH₂)-, -(CH₂)ₘ-NHCO-(CH₂)ₜ-CONH-(CH )ₚ-, -(CH₂)ₘ-NHCONH-(CH₂)ₜ-NHCONH-(CH₂)ₚ- steht,
- R⁶⁰: für Methyl, Ethyl, Benzyl oder Phenyl steht,
- n: für eine ganze Zahl von 1 bis 10 steht,
- m und p: unabhängig voneinander für eine ganze Zahl von 0 bis 4 stehen,
- o: für eine ganze Zahl von 0 bis 2 steht und
- t: für eine ganze Zahl von 1 bis 6 steht,
und mindestens einen UV-Absorber ausgewählt aus der Formel (C) enthält,
worin
- R¹⁰¹: für gegebenenfalls verzweigtes C₁- bis C₂₀-Alkyl steht,
- R¹⁰²: für Wasserstoff oder Cyano steht,
- R¹⁰³: für Wasserstoff oder C₁- bis C₁₂-Alkoxy stehen,
- R¹⁰⁷: zusammen mit R¹⁰⁸ für -(CH₂)₂-, -(CH₂)₃- oder -CH₂-C(CH₃)₂- stehen und
- n: für 1 oder 2 steht.

Insbesondere bevorzugt ist eine erfindungsgemäße elektrochrome Lösung, welche mindestens eine Substanz der Formel (Ia)-(Id) enthält,
worin
- OX₂: für einen Rest der Formeln (II), (IV) oder (V) steht,
worin
- R², R⁴ und R⁸: für eine direkte Bindung zum Brückenglied B stehen,
- R³, R⁵ und R⁹: unabhängig voneinander für Methyl, Ethyl, Propyl, Butyl, Pentyl, Hexyl, Heptyl, Octyl, Benzyl oder Phenyl stehen, oder im Falle der Formeln Ic oder Id ebenfalls für eine direkte Bindung zum Brückenglied B stehen,
- R⁶ und R⁷: gleich sind und für Wasserstoff, Methyl, Methoxy, Chlor, Cyano oder Methoxycarbonyl stehen,
- R¹⁰, R¹¹; R¹², R¹³ und R¹⁴, R¹⁵: unabhängig voneinander für Wasserstoff oder, falls Z¹ eine direkte Bindung bedeutet, jeweils paarweise zusammen für eine -CH=CH-Brücke stehen,
- R⁶⁹ bis R⁷²: gleich sind und Wasserstoff, Methyl oder Ethyl bedeuten,
- R⁷³ und R⁷⁴: Wasserstoff bedeuten,
- E¹ und E²: gleich sind und für O oder S stehen,
- Z¹: für eine direkte Bindung oder -CH=CH- steht,
- X: für ein unter den Bedingungen redox-inertes, farbloses Anion steht,
- RED₁: für einen Rest der Formeln (X), (XII), (XIII), (XVI) oder (XVII) steht,
- R²⁸, R³⁴, R³⁸, R⁴⁶ und R⁴⁹: für eine direkte Bindung zum Brückenglied B stehen,
- R²⁹ bis R³¹, R³⁵ und R³⁹: unabhängig voneinander für Methyl, Ethyl, Propyl, Butyl, Pentyl, Hexyl, Heptyl, Octyl, Benzyl oder Phenyl stehen, oder im Falle der Formeln Ib oder Id R³⁰, R³⁵ und R³⁹ ebenfalls für die direkte Bindung zum Brückenglied B stehen,
- R³², R⁴⁷ und R⁴⁸: für Wasserstoff stehen,
- R³⁶, R³⁷, R⁴⁰, R⁴¹ und R⁵⁰ bis R⁵²: unabhängig voneinander für Wasserstoff, Methyl, Methoxy, Chlor, Cyan, Methoxycarbonyl oder Phenyl stehen, oder im Falle der Formeln Ib oder Id R⁵¹ ebenfalls für eine direkte Bindung zum Brückenglied B steht,
- Z³: für eine direkte Bindung, eine -CH=CH- oder -N=N-Brücke steht,
- =Z⁴=: für eine direkte Doppelbindung, eine =CH-CH= oder =N-N=-Brücke steht,
- E³ bis E⁵: unabhängig voneinander für O, S oder NR⁵⁹ stehen, E³ und E⁴ aber die gleiche Bedeutung haben,
- E⁶ bis E⁹: untereinander gleich sind und für S, Se oder NR⁵⁹ stehen,
- R⁵⁹: für Methyl, Ethyl, Propyl, Butyl, Pentyl, Hexyl, Heptyl, Octyl, Benzyl oder Phenyl steht, oder im Falle der Formel XVI in Ib oder Id ebenfalls für eine direkte Bindung zum Brückenglied B steht,
- B: für ein Brückenglied der Formeln -(CH₂)ₙ-, -(CH₂)ₘ-O-(CH₂)ₚ-, -(CH₂)ₘ-NR⁶⁰-(CH₂)ₚ-, -(CH₂)ₘ-C₆H₄-(CH₂)ₚ-, -O-(CH₂)ₚ-O-, -NR⁶⁰-(CH₂)ₚ-NR⁶⁰-, -(CH₂)ₘ-OCO-C₆H₄-COO-(CH₂)ₚ-, -(CH₂)ₘ-NHCO-C₆H₄-CONH-(CH₂)ₚ-, -(CH₂)ₘ-NHCONH-C₆H₄-NHCONH-(CH₂)ₚ-, -(CH₂)ₘ-OCO-(CH₂)ₜ-COO-(CH₂)ₚ-,-(CH₂)ₘ-NHCO-(CH₂)ₜ-CONH-(CH₂)ₚ-, -(CH₂)ₘ-NHCONH-(CH₂)ₜ-NHCONH-(CH₂)ₚ-steht,
- R⁶⁰: für Methyl steht,
- n: für eine ganze Zahl von 1 bis 10 steht,
- m und p: gleich sind und für eine ganze Zahl von 0 bis 2 stehen und
- t: für eine ganze Zahl von 1 bis 6 steht,
und mindestens einen UV-Absorber ausgewählt aus der Formel (C) enthält,
worin
- R¹⁰¹: für Methyl, Ethyl, 1- oder 2-Propyl, 1- oder 2-Butyl, 1-Hexyl, 2-Ethyl-1-hexyl, 1-Octyl oder 1-Dodecyl steht,
- R¹⁰²: für Wasserstoff oder Cyano steht,
- R¹⁰³: für Wasserstoff, Methoxy, Ethoxy, Propoxy, Butoxy, Hexoxy oder Octoxy stehen,
- R¹⁰⁷: zusammen mit R¹⁰⁸ für -CH₂-C(CH₃)₂- stehen und
- n: für 1 oder 2 steht.

Ganz besonders bevorzugt ist eine erfindungsgemäße elektrochrome Lösung, welche mindestens eine Substanz der Formel (Ia) entsprechend einer der Formeln 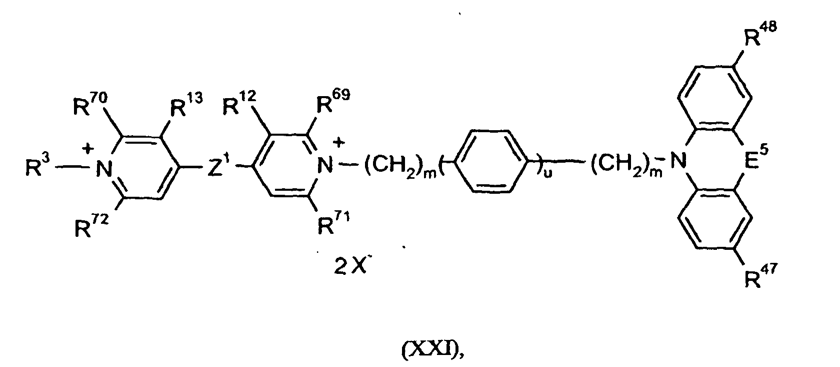 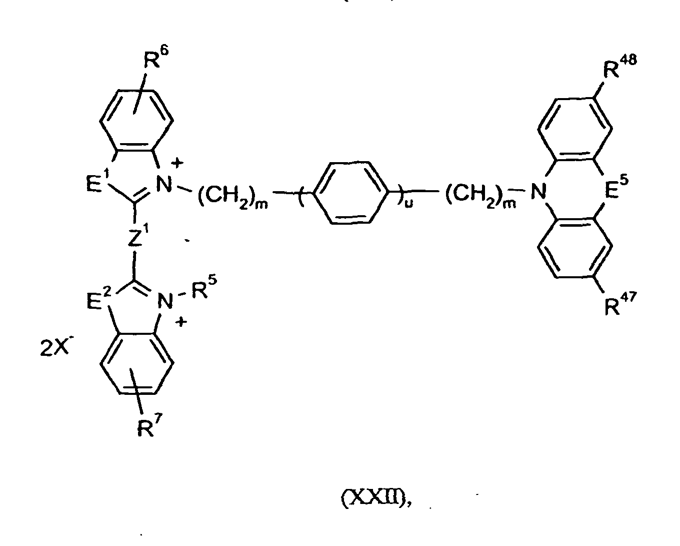 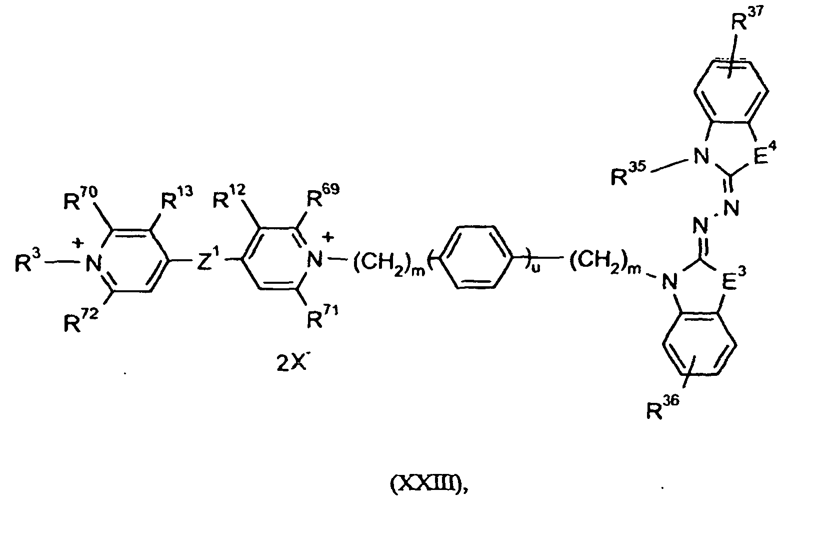 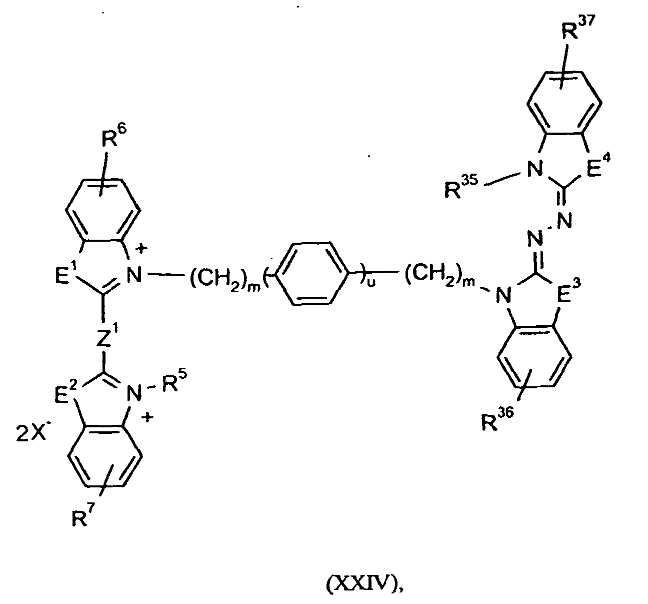 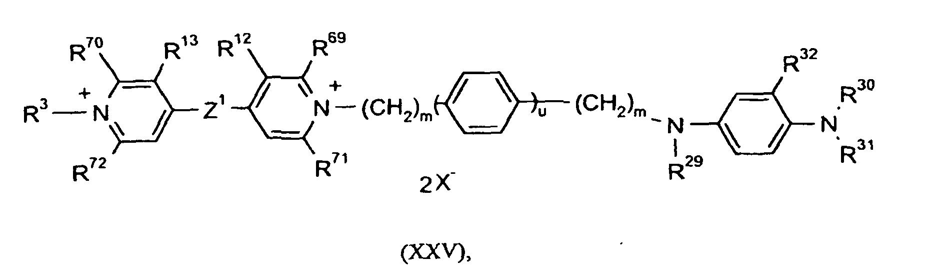 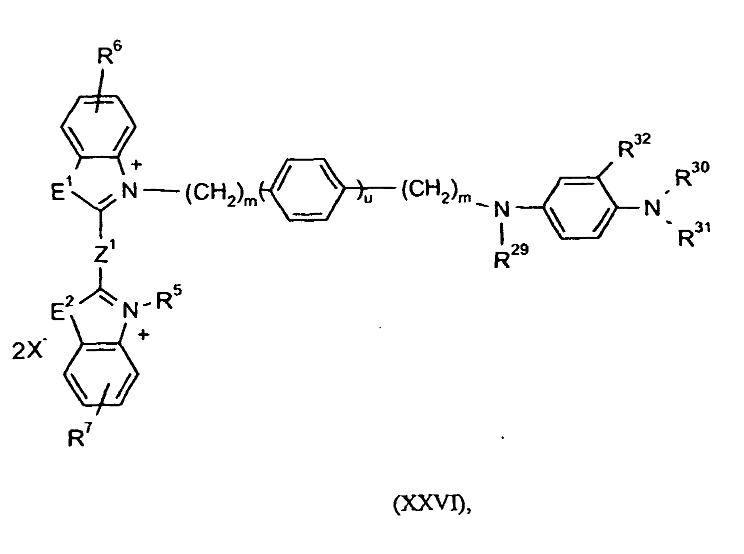 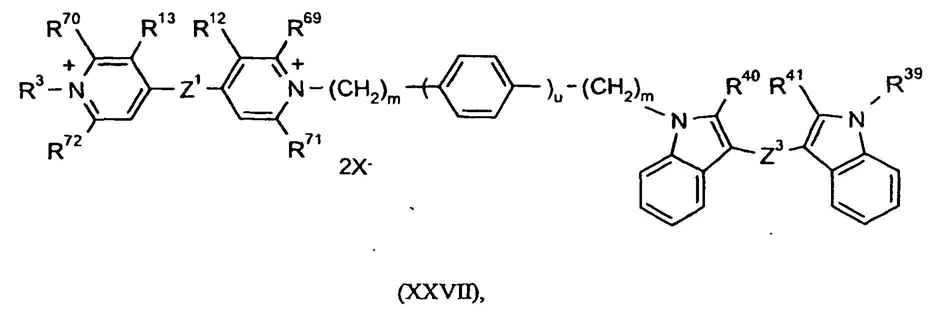 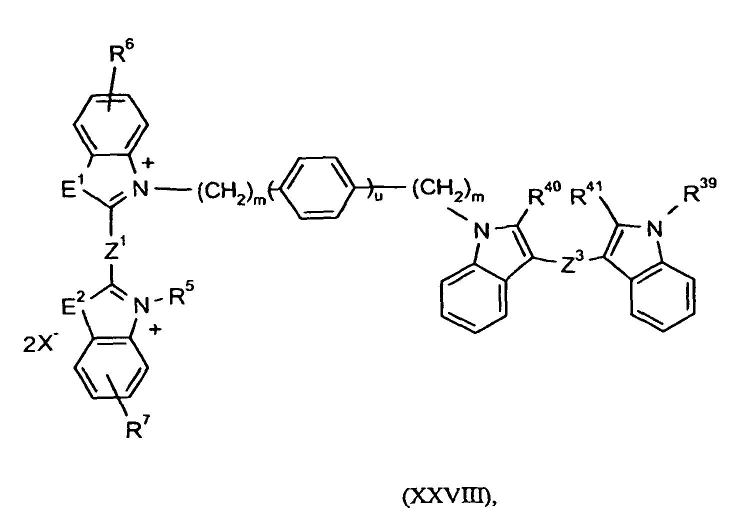 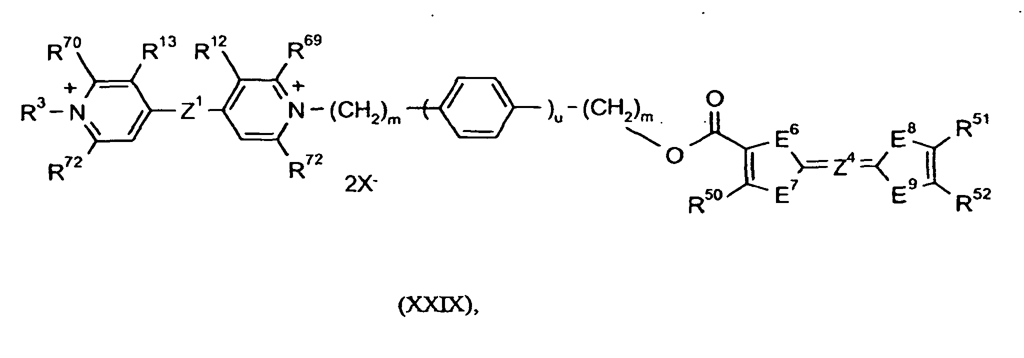 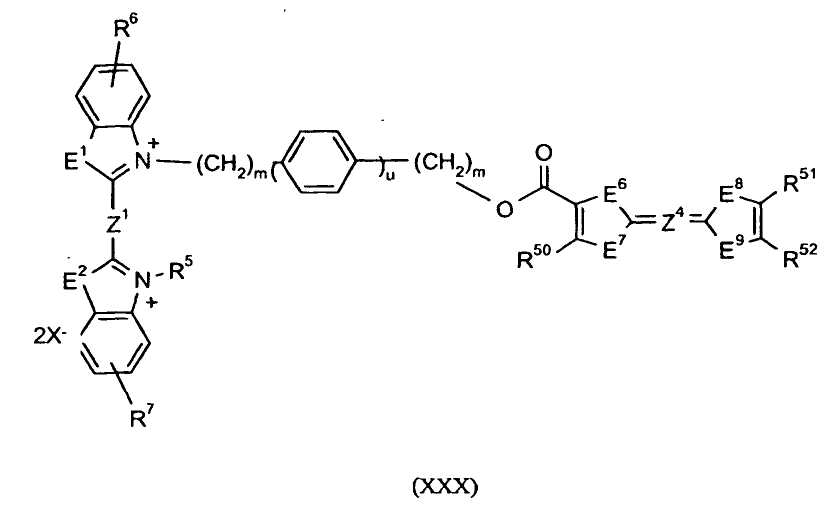 enthält, oder mindestens eine Substanz der Formel (Ib) entsprechend einer der Formeln 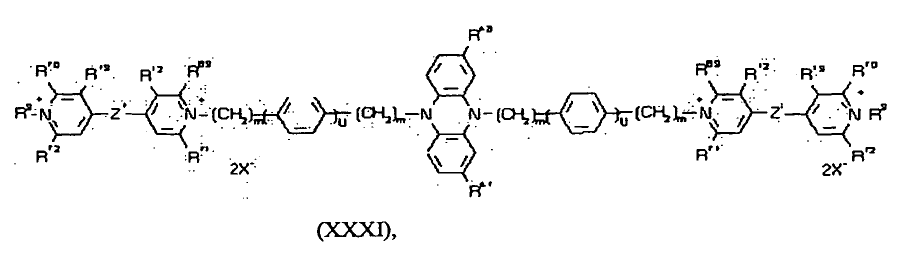 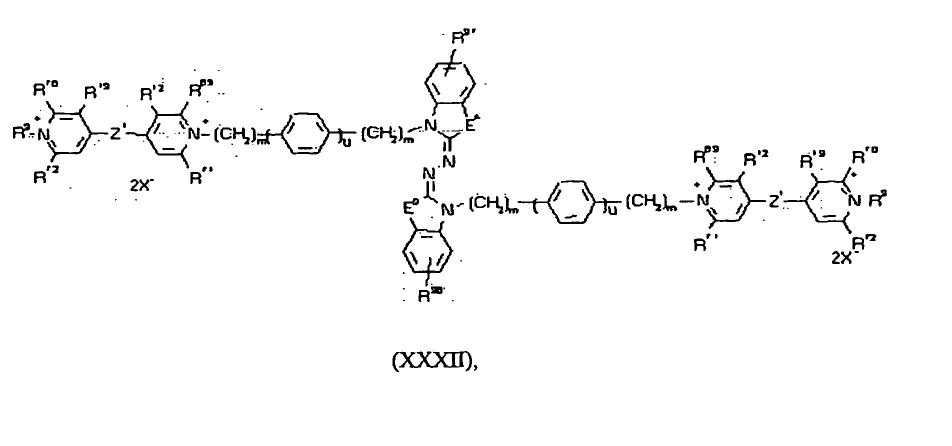 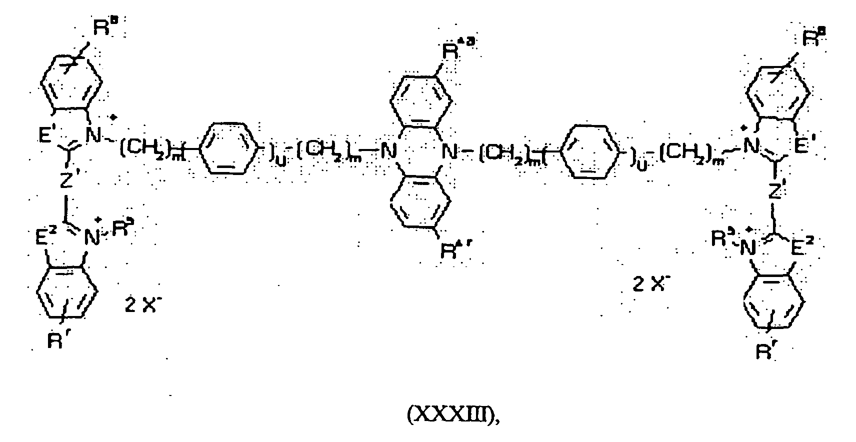 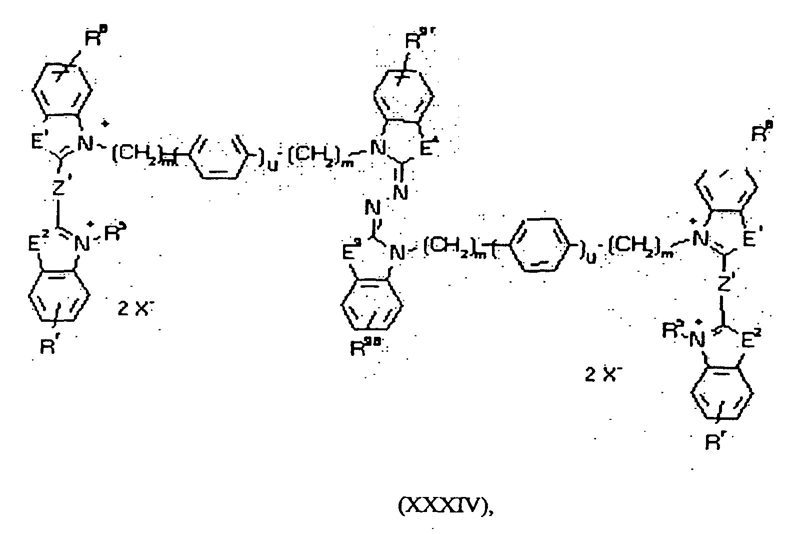 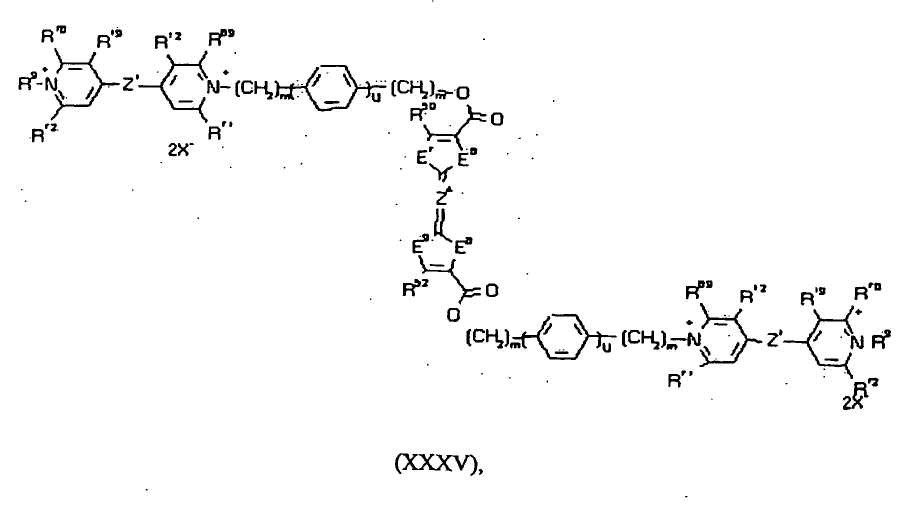 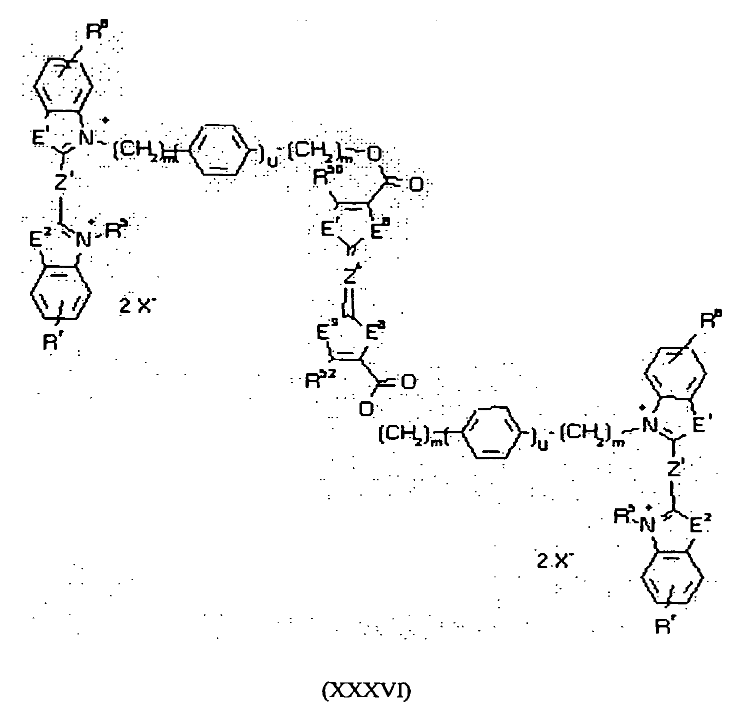 oder mindestens eine Substanz der Formel (Ic) entsprechend einer der Formeln 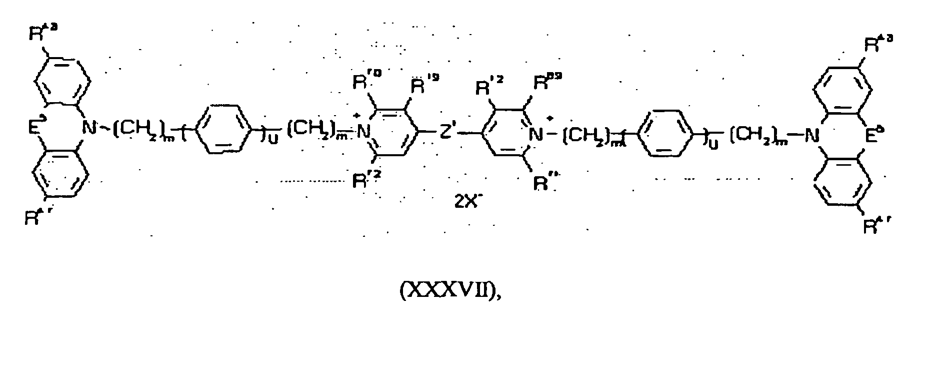 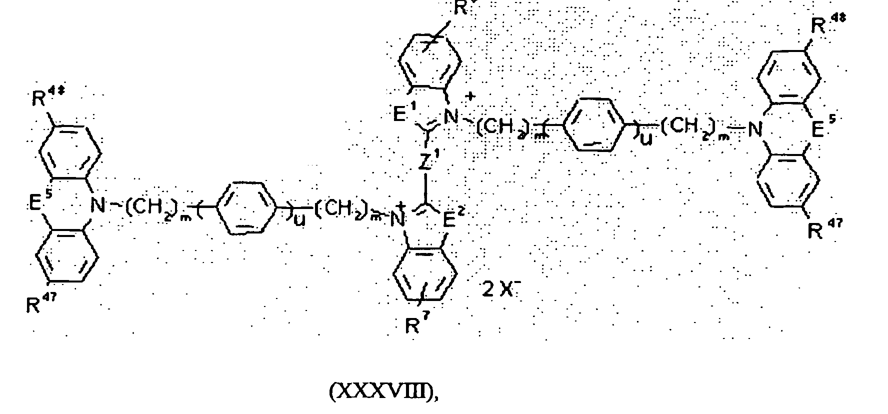 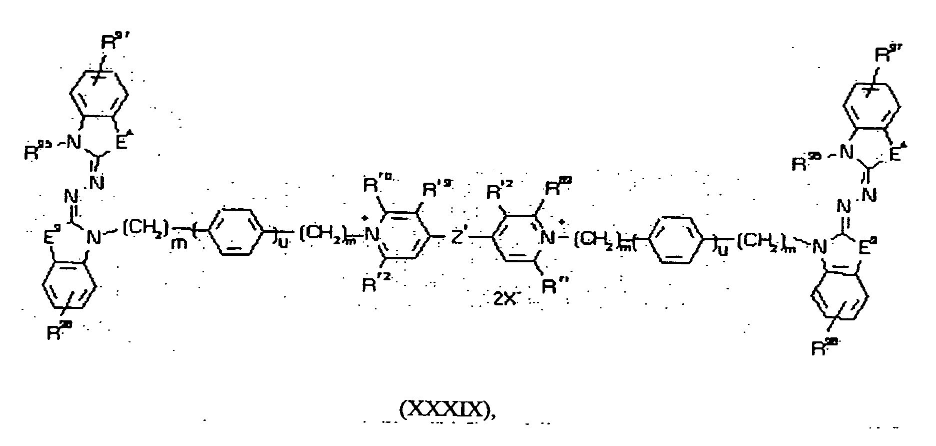 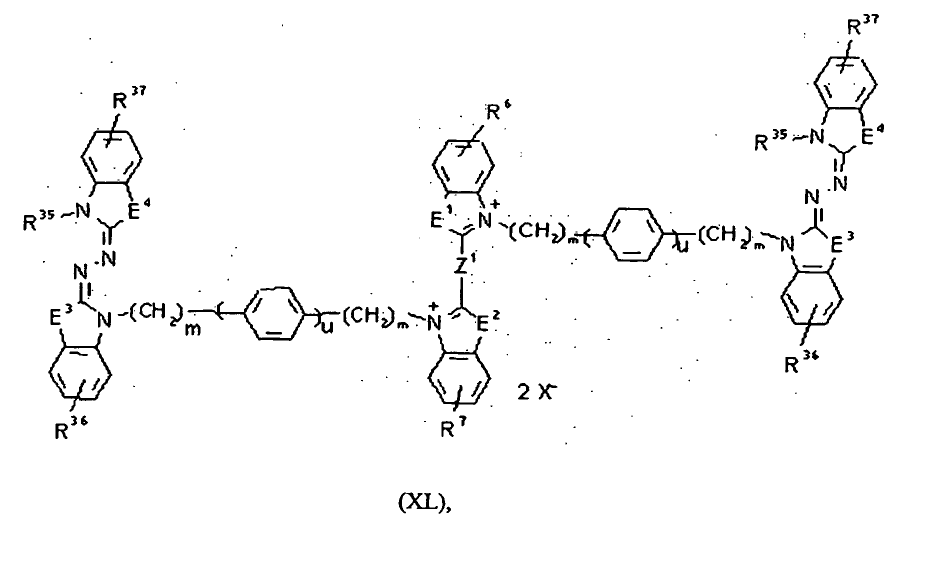 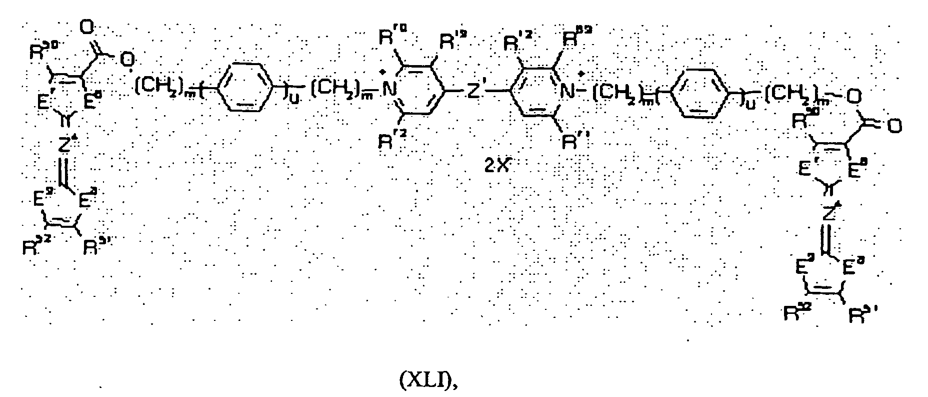 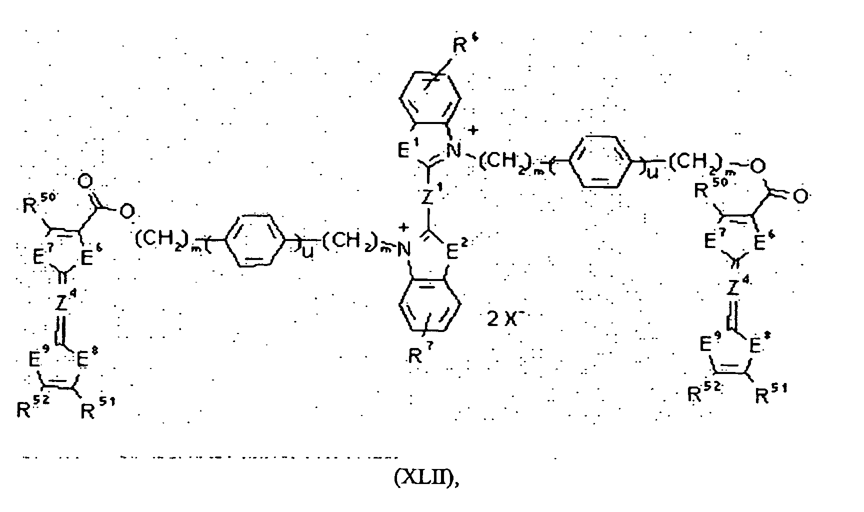 worin
- R³, R⁵, R³⁵ und R³⁹: unabhängig voneinander für Methyl, Ethyl, Propyl, Butyl, Pentyl, Hexyl oder Benzyl stehen,
- R⁶, R⁷ und R³⁶, R³⁷: paarweise gleich sind und für Wasserstoff, Methyl, Methoxy, Chlor, Cyano oder Methoxycarbonyl stehen,
- R¹² und R¹³: für Wasserstoff oder, wenn Z¹ eine direkte Bindung bedeutet, gemeinsam für eine -CH=CH-Brücke stehen,
- R⁶⁹ bis R⁷²: gleich sind und für Wasserstoff oder Methyl stehen,
- E¹ und E²: gleich sind und für O oder S stehen,
- Z¹: für eine direkte Bindung oder -CH=CH- steht,
- R³², R⁴⁷ und R⁴⁸: für Wasserstoff stehen,
- E³ bis E⁵: unabhängig voneinander für O, S oder NR⁵⁹ stehen, wobei E³ und E⁴ aber gleich sind,
- R²⁹ bis R³¹ und R⁵⁹: unabhängig voneinander für Methyl, Ethyl, Propyl, Butyl, Pentyl, Hexyl oder Benzyl stehen, wobei R²⁹ bis R³¹ vorzugsweise gleich sind,
- R⁴⁰ und R⁴¹: gleich sind und für Wasserstoff, Methyl, Ethyl, Propyl, Butyl oder Phenyl stehen,
- Z³: für eine direkte Bindung, -CH=CH- oder -N=N- steht,
- R⁵⁰ bis R⁵²: unabhängig voneinander für Wasserstoff, Methyl, Methoxy, Chlor, Cyano, Methoxycarbonyl, Ethoxycarbonyl oder Phenyl stehen, vorzugsweise jedoch gleich sind,
- E⁶ bis E⁹: untereinander gleich sind und für S, Se oder NR⁵⁹ stehen,
- Z⁴: für eine direkte Doppelbindung, eine =CH-CH= oder =N-N=Brücke steht,
- m: für eine ganze Zahl von 1 bis 5 steht,
- u: für 0 oder 1 steht und
- X⁻: für ein unter den Bedingungen redox-inertes, farbloses Anion steht,
und einen UV-Absorber der Formel (C),
worin
- R¹⁰¹: für Ethyl oder 2-Ethyl-1-hexyl steht,
- R¹⁰²: für Wasserstoff steht,
- R¹⁰³: für m- und/oder p-ständiges Methoxy oder Ethoxy steht,
- R¹⁰⁷: zusammen mit R¹⁰⁸ für -CH₂-C(CH₃)₂- stehen und
- n: für 1 oder 2 steht,
enthält.

Ganz besonders bevorzugt ist eine erfindungsgemäße elektrochrome Lösung, die einen UV-Absorber der Formel

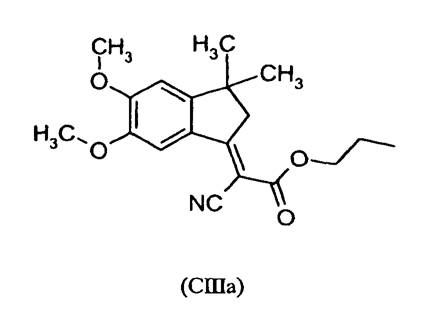

Die Substanzen der Formel (I) sind bekannt aus WO 97/30134.

In den oben genannten Substituentenbedeutungen sind Alkylreste, auch abgewandelte, wie z.B. Alkoxy- oder Aralkylreste, vorzugsweise solche mit 1 bis 12 C-Atomen, insbesondere mit 1 bis 8 C-Atomen, sofern nichts anderes angegeben ist. Sie können geradkettig oder verzweigt sein und gegebenenfalls weitere Substituenten tragen wie z.B. C₁- bis C₄-Alkoxy, Fluor, Chlor, Hydroxy, Cyano, C₁- bis C₄-Alkoxycarbonyl oder COOH.

Unter Cycloalkylresten werden vorzugsweise solche mit 3 bis 7 C-Atomen, insbesondere 5 oder 6 C-Atomen verstanden.

Alkenylreste sind vorzugsweise solche mit 2 bis 8 C-Atomen, insbesondere 2 bis 4 C-Atomen.

Arylreste, auch solche in Aralkylresten, sind vorzugsweise Phenyl- oder Naphthylreste, insbesondere Phenylreste. Sie können durch 1 bis 3 der folgenden Reste substituiert sein: C₁- bis C₆-Alkyl, C₁- bis C₆-Alkoxy, Fluor, Chlor, Brom, Cyano, Hydroxy, C₁- bis C₆-Alkoxycarbonyl oder Nitro. Zwei benachbarte Reste können auch einen Ring bilden.

Die erfindungsgemäße elektrochrome Lösung enthält mindestens ein Lösungsmittel. Geeignete Lösungsmittel sind alle unter den gewählten Spannungen redox-inerten Lösungsmittel, die keine Elektrophile oder Nukleophile abspalten können oder selber als ausreichend starke Elektrophile oder Nukleophile reagieren und so mit den farbigen Radikalionen reagieren könnten. Beispiele sind Propylencarbonat, γ-Butyrolacton, Acetonitril, Propionitril, Giutaronitril, Methylglutarnitril, 3,3'-Oxydipropionitril, Hydroxypropionitril, Dimethylforrnamid, N-Methylpyrrolidon, Sulfolan, 3-Methylsulfolan oder Mischungen davon. Bevorzugt sind Propylencarbonat und Mischungen davon mit Glutaronitril oder 3-Methylsulfolan.

Die erfindungsgemäße elektrochrome Lösung kann mindestens ein inertes Leitsalz enthalten.

Als inerte Leitsalze sind Lithium-, Natrium- und Tetraalkylammoniumsalze geeignet, insbesondere letztere. Die Alkylgruppen können zwischen 1 und 18 C-Atome aufweisen und gleich oder verschieden sein. Bevorzugt ist Tetrabutylammonium.

Als Anionen zu diesen Salzen, aber auch als Anionen X⁻ in den Formeln (I), (II), (IV), (VI) und (VII) kommen alle redox-inerten, farblosen Anionen in Frage. Beispiele sind Tetrafluoroborat, Tetraphenylborat, Cyano-triphenylborat, Perchlorat, Chlorid, Nitrat, Sulfat, Phosphat, Methansulfonat, Ethansulfonat, Tetradecansulfonat, Pentadecansulfonat, Trifluormethansulfonat, Perfluorbutansulfonat, Perfluoroctansulfonat, Benzolsulfonat, Chlorbenzolsulfonat, Toluolsulfonat, Butylbenzolsulfonat, tert. Butylbenzolsulfonat, Dodecylbenzolsulfonat, Naphthalinsulfonat, Biphenylsulfonat, Benzoldisulfonat, Naphthalindisulfonat, Biphenyldisulfonat, Nitrobenzolsulfonat, Dichlorbenzolsulfonat, Trifluormethylbenzolsutfonat, Hexafluorophosphat, Hexafluoroarsenat, Hexafluorosilicat, 7,8- oder 7,9-Dicarba-nido-undecaborat(1-) oder (2-), die gegebenenfalls an den B- und/oder C-Atomen durch eine oder zwei Methyl-, Ethyl, Butyl- or Phenyl-Gruppen substituiert sind sowie Dodecahydro-dicarbadodecaborat(2-) oder B-Methyl-C-phenyl-dodecahydro-dicarbadodecaborat(1-). Bei mehrwertigen Anionen steht X⁻ für ein Äquivalent dieses Anions, z.B. für 1/2 SiF₆²⁻.

Bevorzugte Anionen sind Tetrafluoroborat, Pentadecansulfonat, Dodecylbenzolsulfonat, Cyano-triphenylborat, 7,8-Dicarba-nido-undecaborat(1-).

Die Leitsalze werden beispielsweise im Bereich 0 bis 1 molar eingesetzt.

Als weitere Zusätze zu der elektrochromen Lösung können Verdicker eingesetzt werden, um die Viskosität der Flüssigkeit zu steuern. Das kann Bedeutung haben zur Vermeidung von Segregation, d.h. der Bildung von streifiger oder fleckiger Farbbildung bei längerem Betrieb einer die erfmdungsgernäße elektrochrome Flüssigkeit enthaltenden elektrochromen Vorrichtung im eingeschalteten Zustand, und zur Steuerung der Ausbleichgeschwindigkeit nach Abschalten des Stroms.

Als Verdicker eignen sich alle für diese Zwecke üblichen Verbindungen wie z.B. Polyacrylat, Polymethacrylat (Luctite L®),Polycarbonat und Polyurethan.

Die elektrochrome Lösung kann auch gelförmig sein.

Erfindungsgemäßer Bestandteil der erfindungsgemäßen elektrochromen Lösung sind UV-Absorber. Sie werden im Bereich 0,01 bis 2 mol/l, vorzugsweise 0,04 bis 1 mol/l eingesetzt. Die in der erfindungsgemäßen Lösung enthaltenen UV-Absorber sind im Prinzip bekannt oder können analog zu der Herstellung der bekannten UV-Absorber hergestellt werden. UV-Absorber sind die der Formeln (C) bzw. (CIIIa). Diese sind in den genannten Lösungsmitteln sehr gut löslich, z.B. in Propylencarbonat mindestens 0,8 molar.

Die Wirkung der UV-Absorber wurde gemessen in elektrochromen Zellen, wie sie weiter unten beschrieben sind. Als Belichtungsgerät wurde Xenotest 150 S der Fa. Heraeus verwendet. Die Leistung betrug 1570 W/m² in der Konfiguration "Sonnenlicht im Freien".

Überraschenderweise wurde gefunden, dass Mischungen dieser UV-Absorber erheblich wirkungsvoller sind als die einzelnen Substanzen.

Die erfindungsgemäße elektrochrome Lösung enthält die Substanzen der Formel (I), insbesondere der Formeln (Ia) bis (Id), jeweils in einer Konzentration von mindestens 10⁻⁴ mol/l, vorzugsweise 0,001 bis 1 mol/l. Es können auch Mischungen mehrerer elektrochromer Substanzen der Formel (I) eingesetzt werden.

Die erfindungsgemäßen elektrochromen Lösungen sind bestens als Bestandteil einer elektrochromen Vorrichtung geeignet. In einer elektrochromen Vorrichtung dient die erfindungsgemäße elektrochrome Lösung als Medium mit variabler Transmission, d.h. unter Einfluss einer elektrischen Spannung ändert sich die Lichtdurchlässigkeit der Lösung, indem sie von einem farblosen in einen gefärbten Zustand übergeht. Weiterer Gegenstand der vorliegenden Erfindung sind demnach elektrochrome Vorrichtungen enthaltend eine erfindungsgemäße elektrochrome Lösung. Der Aufbau einer elektrochromen Vorrichtung, die z.B. als Fensterscheibe, Autosonnendach, Automobil-Rückspiegel oder Display ausgebildet sein kann, ist im Prinzip bekannt. Die erfindungsgemäße elektrochrome Vorrichtung besteht aus zwei einander zugewandten, lichtdurchlässigen Glas- oder Kunststoffscheiben, von denen gegebenenfalls eine verspiegelt ist und deren einander zugewandten Seiten elektrisch leitfähig beschichtet sind, z.B. mit Indium-Zinn-Oxid (ITO), zwischen denen sich die erfindungsgemäße elektrochrome Flüssigkeit befindet. Als leitfähige Materialien sind auch geeignet: Antimon-dotiertes Zinnoxid, Fluor-dotiertes Zinnoxid, Antimon-dotiertes Zinkoxid, Aluminiumdotiertes Zinkoxid, Zinnoxid; auch leitfähige organische Polymere wie gegebenenfalls substituierte Polythienyle, Polypyrrole, Polyaniline, Polyacetylen. Im Falle, dass eine der Scheiben verspiegelt ist, kann auch diese als leitfähige Schicht genutzt werden. Der Abstand der beiden Scheiben beträgt im Allgemeinen 0,005-2 mrn, vorzugsweise 0,02-0,5 mm. Der gewünschte Abstand zwischen den Scheiben wird im Allgemeinen durch einen Dichtungsring hergestellt.

Solche Zellen wurden auch zur Bestimmung der Wirkung der UV-Absorber (s.o.) eingesetzt.

Im Falle, dass die elektrochrome Vorrichtung eine elektrochrome Anzeigevorrichtung ist, sind mindestens eine der beiden leitfähigen Schichten beziehungsweise beide in elektrisch voneinander getrennte Segmente aufgeteilt, die einzeln kontaktiert sind.

Es kann aber auch nur eine der beiden Platten leitfähig beschichtet und in Segmente aufgeteilt sein. Die Trennung der Segmente kann beispielsweise erfolgen durch mechanisches Entfernen der leitfähigen Schicht beispielsweise durch Ritzen, Kratzen, Schaben oder Fräsen oder auf chemischem Wege beispielsweise durch Ätzen mittels beispielsweise einer salzsauren Lösung von FeCl₂ und SnCl₂. Diese Entfernung der leitfähigen Schicht kann über Masken, z. B. solchen aus Photolack, örtlich gesteuert werden. Es können aber auch die elektrisch getrennten Segmente durch gezieltes, z.B. mittels Masken, Aufbringen, z.B. Sputtern oder Drucken, der leitfähigen Schicht hergestellt werden. Die Kontaktierung der Segmente erfolgt beispielsweise mittels feiner Streifen aus leitfähigem Material, womit das Segment mit einem Kontakt am Rande der elektrochromen Vorrichtung elektrisch leitend verbunden ist. Diese feinen Kontaktstreifen können entweder aus dem gleichen Material bestehen wie die leitfähige Schicht selbst und beispielsweise bei deren Aufteilung in Segmente wie oben beschrieben mit hergestellt werden. Sie können aber auch z. B. zur Verbesserung der Leitfähigkeit aus anderem Material wie feinen metallischen Leitern, beispielsweise aus Kupfer oder Silber, bestehen. Auch eine Kombination aus metallischem Material und dem Material der leitfähigen Beschichtung ist möglich. Diese metallischen Leiter können beispielsweise entweder in feiner Drahtform aufgebracht, z.B. aufgeklebt, werden oder aber aufgedruckt werden. Alle diese eben beschriebnenen Techniken sind im Allgemeinen aus der Herstellung von Flüssigkristalldisplays (LCD) bekannt.

Die Anzeigen können im Durchlicht oder auch reflektiv über eine Verspiegelung betrachtet werden.

Die beiden Platten werden mit den einander zugewandten leitfähig beschichteten und in Segmente aufgeteilten Seiten, getrennt durch beispielsweise einen Dichtungsring, aufeinander gelegt und am Rand miteinander verklebt. Der Dichtungsring kann beispielsweise aus Kunststoff oder Dünnglas oder einem anderen gegenüber der elektrochromen Flüssigkeit inerten Material bestehen. Der Abstand zwischen den Platten kann aber auch hergestellt werden mittels anderer Abstandshalter, beispielsweise kleiner Kunststoff- oder Glaskügelchen oder bestimmter Sandfraktionen, wobei dann diese Abstandshalter zusammen mit dem Kleber aufgetragen werden und dann gemeinsam den Dichtungsring bilden. Der Dichtungsring enthält eine oder zwei Aussparungen, die zum Befüllen der elektrochromen Vorrichtung dienen. Der Abstand zwischen den beiden Platten liegt zwischen 0.005 und 2 mm, vorzugsweise 0.02 bis 0.5 mm. Bei großflächigen Anzeigevorrichtungen, insbesondere solchen aus Kunststoff, kann es vorteilhaft sein, mittels Abstandshaltern, beispielsweise Kunststoffkügelchen gleichen Durchmessers, die über die Fläche der Anzeigevorrichtung verteilt sind, den Abstand der Platten konstant zu halten.

Diese Anzeigevorrichtung wird mit einer elektrochromen Flüssigkeit befüllt über die Öffnungen im Dichtungsring, wobei stets unter Feuchtigkeits- und Sauerstoffausschluss gearbeitet werden muss. Das Befüllen kann beispielsweise mittels feiner Kanülen oder aber über die Vakuumbefülltechnik erfolgen, bei der die Vorrichtung und die Flüssigkeit, eingefüllt in eine flache Schale, in einen evakuierbaren Behälter eingebracht werden. Dieser wird evakuiert. Dann wird die Anzeigevorrichtung, die nur eine Befüllöffnung enthält, mit dieser Öffnung in die Flüssigkeit getaucht. Beim Entfernen des Vakuums wird nun die Flüssigkeit in die Anzeigevorrichtung gedrückt.

Die erfindungsgemäße selbstlöschende einzellige elektrochrome Vorrichtung kann zusätzlich zu den oben beschriebenen elektrochromen Substanzen der Formeln (I), insbesondere den Formeln (Ia) bis (Id), auch andere enthalten, wie sie beispielsweise in US-P 4,902,108, Topics in Current Chemistry, Vol. 92, S. 1-44 (1980) und Angew. Chem. 90, 927 (1978) beschrieben sind. Solche elektrochromen Substanzen entstammen beispielsweise den oben unter den Formeln (II) bis (XX) angegebenen Gruppen, wobei dann keiner der angeführten Reste die Bedeutung "direkte Bindung zur Brücke B" besitzen kann. Andere geeignete elektrochrome Mischkomponenten sind beispielsweise Tetrazoliniumsalze oder Salze oder Komplexe von Metallionen, z.B. [Fe(C₅H₅)₂]^{0/1+}. Eine Zumischung solcher Redoxsysteme kann beispielsweise vorteilhaft sein, um bei der erfindungsgemäßen elektrochromen Vorrichtung den Farbton, z.B. des Displays, im eingeschalteten Zustand zu korrigieren oder zu intensivieren.

### Beispiele

### Beispiel 1

### Herstellung einer elektrochromen Substanz der Formel (I)

a) Unter Stickstoffatmosphäre wurden 9,2 g Phenazin in 60 ml wasserfreiem Tetrahydrofuran suspendiert. Während 15 min tropften 30,8 ml 20 gew.-%ige Phenyllithiumlösung in Cyclohexan/Diethylether 7 : 3 dazu, wobei die Temperatur bei max. 35°C gehalten wurde. Die Lösung wurde 30 min bei Raumtemperatur nachgerührt.
   Bei 15°C wurden in einer Portion 30,2 ml 1,4-Dibrombutan dazugegeben. Die Temperatur stieg dabei bis 38°C an. Nach 6 h bei Raumtemperatur wurde mit 200 ml Wasser versetzt und der pH auf 7,0 gestellt. Die organische Phase wurde abgetrennt, dreimal mit je 100 ml Wasser gewaschen und im Vakuum eingeengt. Schließlich wurde überschüssiges 1,4-Dibrombutan bei einem Druck von 0,2 mbar abdestilliert. Der ölige Rückstand wurde in 400 ml Ethanol heiß gelöst. Das nach dem Erkalten ausgefallene Produkt wurde abgesaugt, mit Ethanol und Hexan gewaschen und getrocknet. Man erhielt 8,0 g (41 % d. Th.) blassgelbes Pulver des 9,10-Dihydrophenazins der Formel 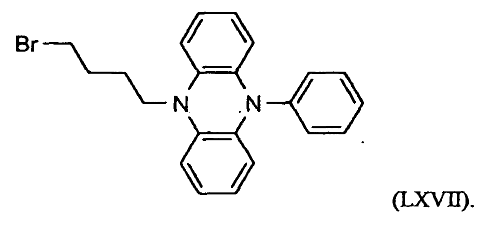
b) 7,5 g des 9,10-Dihydrophenazins der Formel (LXVII) aus a) und 6,1 g 4,4'-Dipyridyl wurden in 100 ml Acetonitril 24 h bei 70°C unter Stickstoffatmosphäre gerührt. Nach dem Abkühlen wurde abgesaugt und mit 50 ml Aceton gewaschen. Nach dem Trocknen erhielt man 6,3 g (60 % d. Th.) des Salzes der Formel 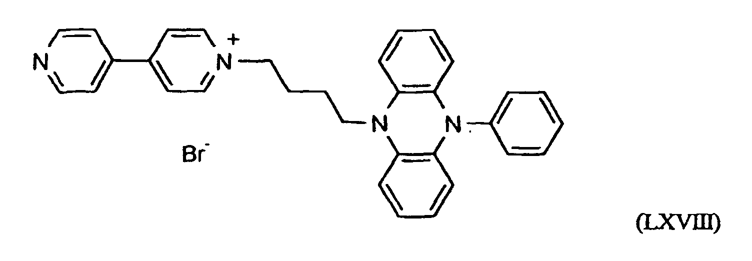
c) 6,1 g des unter b) erhaltenen Salzes wurden in 70 ml N-Methyl-2-pyrrolidon zusammen mit 2,7 ml Benzylbromid 7 h bei 70°C unter Stickstoffatmosphäre gerührt. Nach dem Abkühlen wurde mit 150 ml Toluol verdünnt und das ausgefallene Produkt abgesaugt. Es wurde gründlich mit 150 ml Toluol und 500 ml Hexan gewaschen und getrocknet. Man erhielt 5,5 g (69 % d. Th.) des Dipyridiniumsalzes der Formel 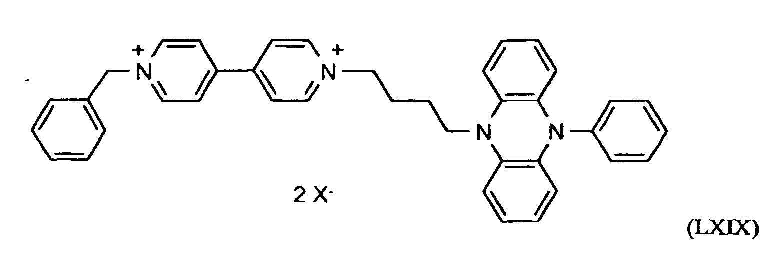 mit X⁻ = Br⁻.
d) 4,0 g dieses Produkts aus c) wurden unter Stickstoffatmosphäre in 100 ml Methanol bei 65°C gelöst. Während 5 min wurden 7,4 g Tetrabutylammoniumtetrafluoroborat eingestreut. Es trat eine Fällung auf. Nach 5 min bei 65°C wurde abgekühlt, abgesaugt, mit 200 ml Methanol und 50 ml Hexan gewaschen und im Vakuum getrocknet. Man erhielt 3,4 g (83 % d. Th.) blaßbeiges Pulver der Formel (LXIX) mit X⁻ = BF₄⁻.

### Beispiel 2

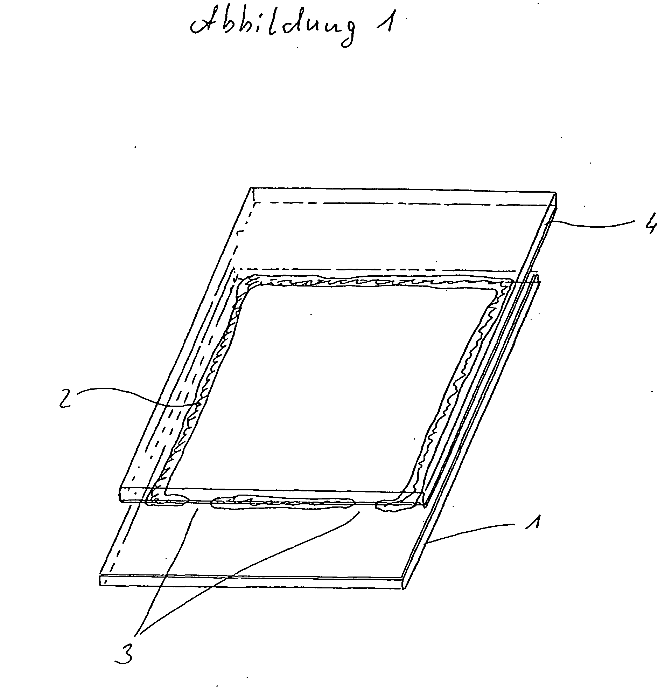

Auf eine mit ITO beschichtete Glasplatte (1) wurde gemäß Abbildung 1 ein Ring (2) aus einer Mischung aus einem Zweikomponenten-Epoxikleber, beispielsweise KÖRAPOX® 735 der Fa. Kömmerling, Pirmasens, und 3 % Glaskugeln vom Durchmesser 200 µm als Abstandshalter aufgetragen, aus dem zwei Einfüllöffnungen (3) ausgespart wurden. Auf diese Kleberaupe wurde eine zweite mit ITO beschichtete Glasplatte (4) mit ihrer Beschichtungsseite aufgelegt. Bei 130°C wurde der Kleber während 10 min ausgehärtet. In einer Glove-box wurde unter Stickstoffatmosphäre eine Lösung eingefüllt, die 0,02 molar an der elektrochromen Verbindung der Formel (LXIX) mit X = BF₄⁻ gemäß Beispiel 1 und 0,1 molar an UV-Absorber der Formel (CIIIa) in wasserfreiem, sauerstofffreiem Propylencarbonat war. Die Befüllöffnungen (3) wurden mit einer Heißklebepistole "Pattex Supermatic" der Firma Henkel KGaA, Düsseldorf, verschlossen. Dieser Klebeauftrag wurde dann mit dem oben beschriebenen Epoxikleber zur mechanischen Sicherung bestrichen und bei Raumtemperatur über Nacht ausgehärtet.

Die Lösung in der Zelle war praktisch farblos. Nach Anlegen einer Spannung von 0,9 V färbte sich die Lösung intensiv grünlich blau mit Maxima bei 466 und 607 nm. Nach Abschalten der Stromzufuhr und Kurzschließen entfärbte sich der Inhalt innerhalb von 10 s wieder.

Es wurden in einem Spektrometer Typ Cary 4G der Fa. Varian die Absorptionskurven im aus- und eingeschalteten (0,9 V) Zustand von 300 bis 800 nm gemessen.

Dann wurde die Zelle im ausgeschalteten Zustand in einem Xenotest 150 S der Fa. Heraeus belichtet. Die Leistung betrug 1570 W/m² in der Konfiguration "Sonnenlicht im Freien". Nach jeweils 7 Tagen (168 h) wurde die Zelle herausgenommen. Es wurden die Geschwindigkeit ("Kinetik") für den Färbe- und Entfärbeprozess und die Spektren wie oben beschrieben im aus- und eingeschalteten Zustand gemessen. Aus den Spektren im ausgeschalteten Zustand wurden Differenzspektren "nach Belichtung" - "vor Belichtung" gebildet und diese bewertet.

Nach 28 Tagen waren die Spektren nahezu unverändert.

### Beispiel 3 (Vergleichsbeispiel)

Es wurde eine Zelle wie in Beispiel 2 beschrieben gebaut, jedoch wurde der UV-Absorber der Formel (CIIIa) weggelassen.

Die Absorptionsmessungen und Belichtung wurden wie in Beispiel 2 durchgeführt. Bereits nach 7 Tagen war eine deutliche Veränderung der Absorption beobachtet.

In den Beispielen 4 bis 5 wurde analog Beispiel 2 vorgegangen. Es wurden jedoch UV-Absorber und ihrer Konzentrationen verändert. Zur Bewertung wurden die Differenzspektren "nach Belichtung" - "vor Belichtung" herangezogen. Die Bewertung erfolgt mit folgenden Noten:
- ++: keine Veränderung
- +: geringe Veränderung
- 0: mäßige Veränderung
- -: deutliche Veränderung
- ―: starke Veränderung, Zelle aber noch funktionstüchtig
leere Felder: keine Messung

| Beispiel | UV-Absorber | Konz. mol/l | 7 Tage | 14 Tage | 21 Tage | 28 Tage | 36 Tage |
|---|---|---|---|---|---|---|---|
| 3 | - | - | - | | | | |
| 2 | (CIIIa) | 0,1 | ++ | ++ | | | |
| 4 | (CIIIa) (CIV) | 0,07 0,07 | ++ | ++ | | | |

wobei es sich bei UV-Absorber der Formel (CIV) um folgende Verbindung handelt:

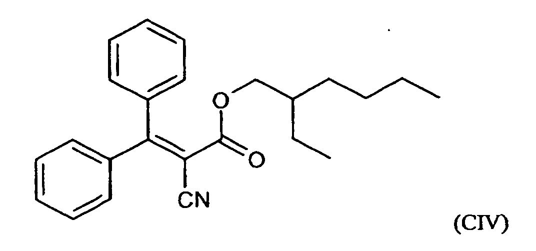

Ganze analoge Ergebnisse wurden mit der elektrochromen Substanz und dem UV-Absorber des Beispiels 5 erzielt.

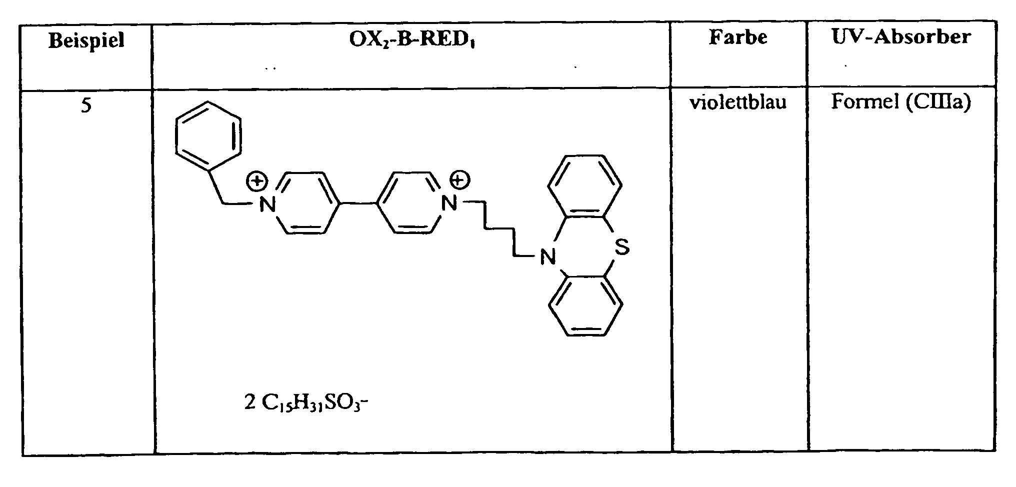

## Patentansprüche

1. UV-geschützte elektrochrome Lösung, enthaltend mindestens eine oxidierbare Substanz RED₁, die durch Elektronenabgabe an einer Anode und mindestens eine reduzierbare Substanz OX₂, die durch Elektronenaufnahme an einer Kathode, unter Zunahme der Extinktion im sichtbaren Bereich des Spektrums von einer schwach gefärbten oder farblosen Form in eine gefärbte Form OX₁ bzw. RED₂ übergeht, wobei nach Ladungsausgleich die schwach gefärbte bzw. farblose Form zurückgebildet wird, **dadurch gekennzeichnet,**
**dass** sie mindestens eine elektrochrome Substanz der Formeln
OX₂-B-RED₁ (Ia),
OX₂-B-RED₁-B-OX₂ (Ib),
RED₁-B-OX₂-B-RED₁ (Ic),
oder
OX₂-(B-RED₁-B-OX₂)_{d}-B-RED₁ (Id),
worin
d für eine ganze Zahl von 1 bis 5 steht,
OX₂ für einen Rest der Formeln 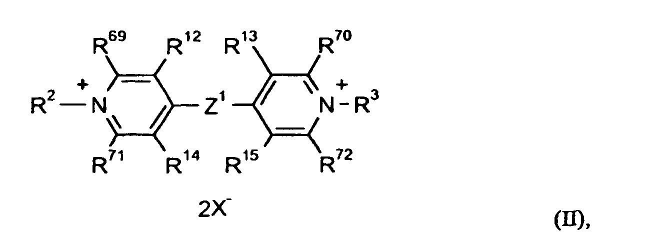 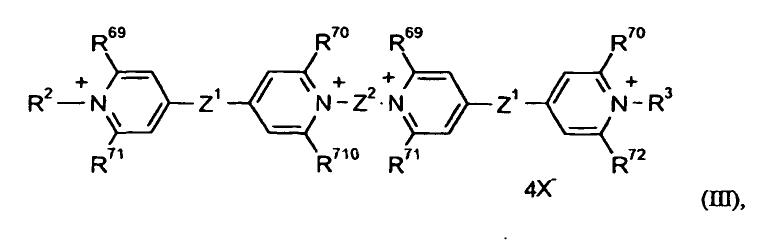 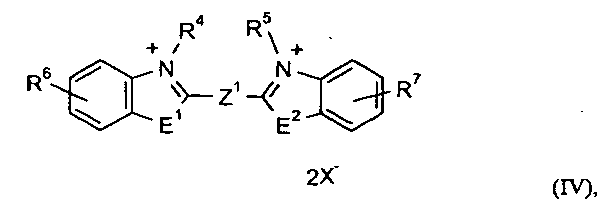 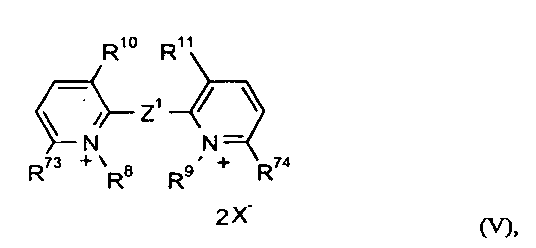 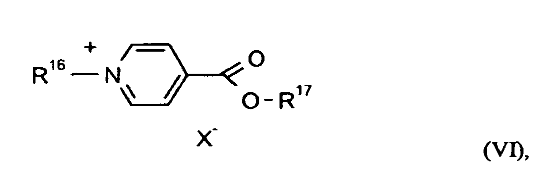 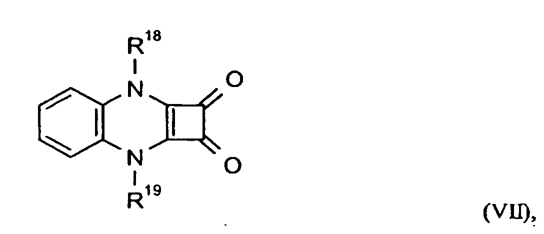 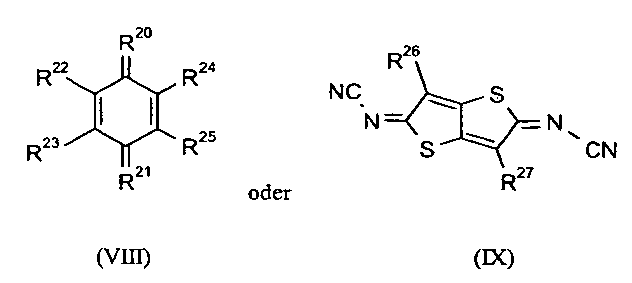
steht, wobei
R² bis R⁵, R⁸, R⁹, R¹⁶ bis R¹⁹ unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten oder
R⁴ und R⁵ oder R⁸ und R⁹ gemeinsam eine -(CH₂)₂- oder -(CH₂)₃-Brücke bilden,
R⁶, R⁷ und R²² bis R²⁵ unabhängig voneinander Wasserstoff, C₁- bis C₄-Alkyl, C₁- bis C₄-Alkoxy, Halogen, Cyano, Nitro oder C₁- bis C₄-Alkoxycarbonyl bedeuten oder
R²² und R²³ und/oder R²⁴ und R²⁵ und R²³ und/oder R²⁴ und R²⁵ eine -CH=CH-CH=CH-Brücke bilden,
R¹⁰ und R¹¹; R¹² und R¹³; R¹⁴ und R¹⁵ unabhängig voneinander Wasserstoff oder paarweise eine -(CH₂)₂-, -(CH₂)₃- oder -CH=CH-Brücke bedeuten,
R⁶⁹ bis R⁷⁴ unabhängig voneinander Wasserstoff oder C₁-C₆-Alkyl bedeuten, oder
R⁶⁹; R¹² und/oder R⁷⁰; R¹³ eine -CH=CH-CH=CH-Brücke bilden,
R²⁰ und R²¹ unabhängig voneinander O, N-CN, C(CN)₂ oder N-C₆- bis C₁₀-Aryl bedeuten,
R²⁶ Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeutet,
E¹ und E² unabhängig voneinander O, S, NR¹ oder C(CH₃)₂ bedeuten oder
E¹ und E² gemeinsam eine -N-(CH₂)₂-N-Brücke bilden,
R¹ C₁- bis C₁₈-Alkyl, C₂- bis C₁₂-Alkenyl, C₄- bis C₇-Cycloalkyl, C₇- bis C₁₅-Aralkyl, C₆- bis C₁₀-Aryl bedeutet,
Z¹ eine direkte Bindung, -CH=CH-, -C(CH₃)=CH-, -C(CN)=CH-, -CCl=CCl-, -C(OH)=CH-, -CCl=CH-, -C=C-, -CH=N-N=CH-, -C(CH₃)=N-N=C(CH₃)- oder -CCl=N-N=CCl- bedeutet,
Z² -(CH₂)ᵣ- oder -CH₂-C₆H₄-CH₂- bedeutet,
r eine ganze Zahl von 1 bis 10 bedeutet und
X⁻ ein unter den Bedingungen redox-inertes Anion bedeutet,
wobei die Bindung zum Brückenglied B über einen der Reste R²-R¹⁹, R²²-R²⁷ oder im Falle, dass E¹ oder E² für NR¹ steht über R¹ erfolgt und die genannten Reste dann für eine direkte Bindung stehen,
RED₁ für einen der folgenden Reste 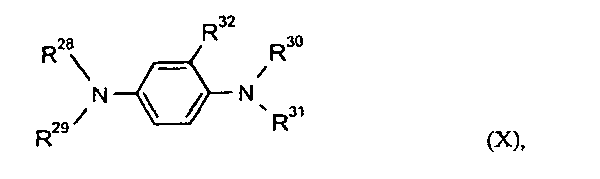 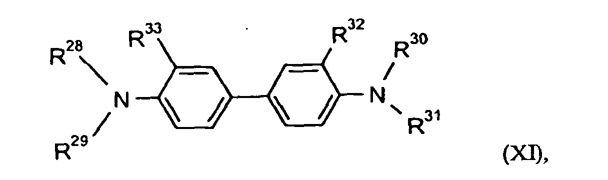 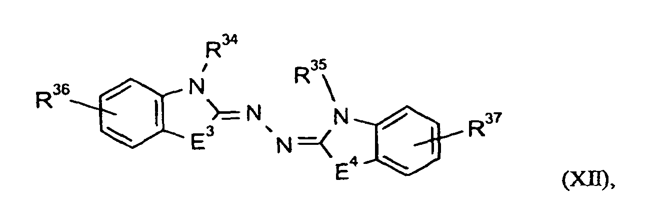 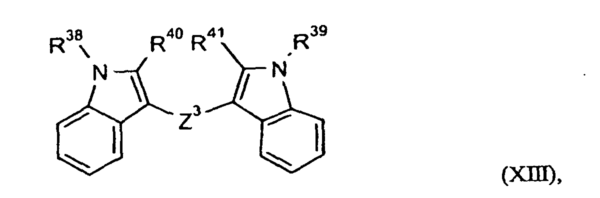 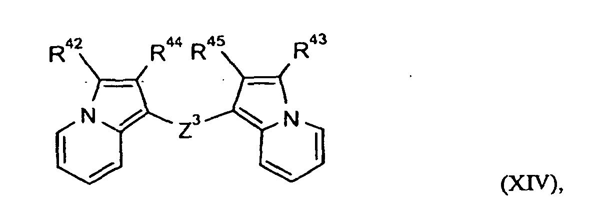 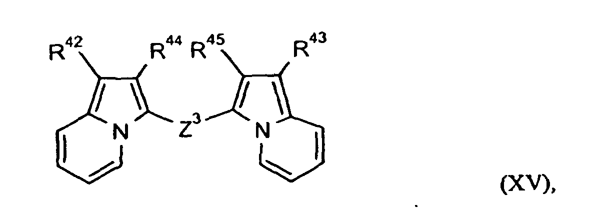 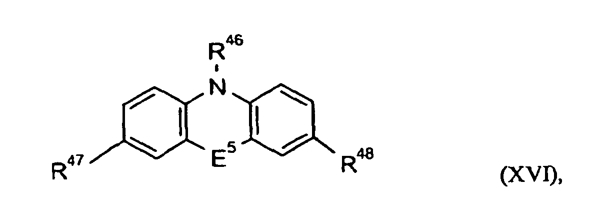 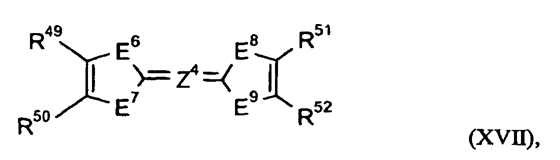 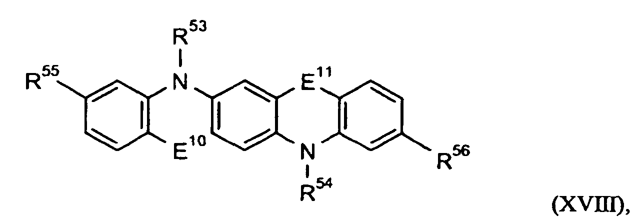 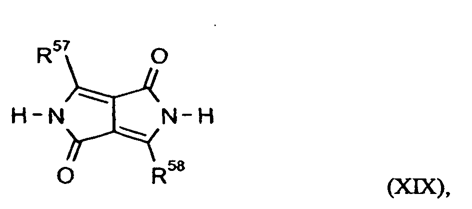 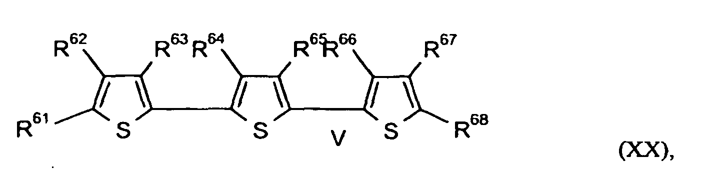
steht, worin
R²⁸ bis R³¹, R³⁴, R³⁵, R³⁸, R³⁹, R⁴⁶, R⁵³ und R⁵⁴ unabhängig voneinander C₁- bis C₁₈-Alkyl, C₂- bis C₁₂-Alkenyl, C₃- bis C₇-Cycloalkyl, C₇- bis C₁₅-Aralkyl oder C₆- bis C₁₀-Aryl bedeuten, und R⁴⁶, R⁵³ und R¹⁴ zusätzlich Wasserstoff bedeuten,
R³², R³³, R³⁶, R³⁷, R⁴⁰, R⁴¹, R⁴² bis R⁴⁵, R⁴⁷, R⁴⁸, R⁴⁹ bis R⁵² und R⁵⁵ bis R⁵⁷ unabhängig voneinander Wasserstoff, C₁- bis C₄-Alkyl, C₁- bis C₄-Alkoxy, Halogen, Cyano, Nitro, C₁- bis C₄-Alkoxycarbonyl oder C₆- bis C₁₀-Aryl bedeuten und R⁵⁷ und R⁵⁸ zusätzlich einen gegebenenfalls benzanellierten aromatischen oder quasiaromatischen fünf- oder sechsgliedrigen heterocyclischen Ring bedeuten und R⁴⁸ zusätzlich NR⁷⁵R⁷⁶ bedeutet,
R⁴⁹ und R⁵⁰ und/oder R⁵¹ und R⁵² eine -(CH₂)₃-, -(CH₂)₄-, -(CH₂)₅- oder -CH=CH-CH=CH-Brücke bilden,
Z³ eine direkte Bindung, eine -CH=CH- oder -N=N-Brücke bedeutet,
=Z⁴= eine direkte Doppelbindung, eine =CH-CH= oder =N-N=-Brücke bedeutet,
E³ bis E⁵, E¹⁰ und E¹¹ unabhängig voneinander O, S, NR⁵⁹ oder C(CH₃)₂ bedeuten und E⁵ zusätzlich C = O oder SO₂ bedeutet, oder
E³ und E⁴ unabhängig voneinander -CH=CH- bedeuten,
E⁶ bis E⁹ unabhängig voneinander S, Se oder NR⁵⁹ bedeuten,
R⁵⁹, R⁷⁵ und R⁷⁶ unabhängig voneinander C₁- bis C₁₂-Alkyl, C₂- bis C₈-Alkenyl, C₃- bis C₇-Cycloalkyl, C₇- bis C₁₅-Aralkyl oder C₆- bis C₁₀-Aryl bedeuten und R⁷⁵ zusätzlich Wasserstoff bedeutet oder
R⁷⁵ und R⁷⁶ in der Bedeutung von NR⁷⁵R⁷⁶ gemeinsam mit dem N-Atom; an das sie gebunden sind, einen fünf- oder sechsgliedrigen, gesättigten Ring bilden, der weitere Heteroatome enthalten kann,
R⁶¹ bis R⁶⁸ unabhängig voneinander Wasserstoff, C₁- bis C₆-Alkyl, C₁- bis C₄-Alkoxy, Cyano, C₁- bis C₄-Alkoxycarbonyl oder C₆- bis C₁₀-Aryl bedeuten, oder
R⁶¹; R⁶² und R⁶⁷, R⁶⁸ unabhängig voneinander gemeinsam eine -(CH₂)₃-, -(CH₂)₄- oder -CH=CH-CH=CH-Brücke bilden,
v eine ganze Zahl zwischen 0 und 10 bedeutet,
wobei die Bindung zum Brückenglied B über einen der Reste R²⁸-R⁵⁸, R⁶¹, R⁶², R⁶⁷, R⁶⁸ oder im Falle, dass einer der Reste E³-E¹¹ für NR⁵⁹ steht über R⁵⁹ erfolgt und die genannten Reste dann für eine direkte Bindung stehen, und
B für ein Brückenglied der Formeln -(CH₂)ₙ- oder -[Y¹ₛ(CH₂)ₘ-Y²]ₒ-(CH₂)ₚ-Y³_{q}-steht, welche jeweils gegebenenfalls durch C₁- bis C₄-Alkoxy, Halogen oder Phenyl substituiert sind,
Y¹ bis Y³ unabhängig voneinander für O, S, NR⁶⁰, COO, CONH, NHCONH, Cyclopentandiyl, Cyclohexandiyl, Phenylen oder Naphthylen stehen,
R⁶⁰ C₁- bis C₆-Alkyl, C₂- bis C₆-Alkenyl, C₄- bis C₇-Cycloalkyl, C₇- bis C₁₅-Aralkyl oder C₆- bis C₁₀-Aryl bedeutet,
n eine ganze Zahl von 1 bis 12 bedeutet,
m und p unabhängig voneinander eine ganze Zahl von 0 bis 8 bedeuten,
o eine ganze Zahl von 0 bis 6 bedeutet und
q und s unabhängig voneinander 0 oder 1 bedeuten,
und einen UV-Absorber der Formel 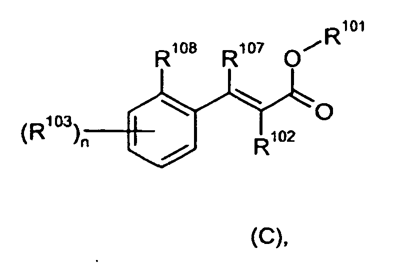 enthält,
worin
R¹⁰¹ für gegebenenfalls verzweigtes C₁- bis C₂₀-Alkyl steht,
R¹⁰² für Wasserstoff, Cyano oder COOR¹ steht,
R¹⁰³ für Wasserstoff, C₁-bis C₁₂-Alkyl oder C₁-bis C₁₂-Alkoxy steht,
R¹⁰⁷ gemeinsam mit R¹⁰⁸ eine C₂- oder C₃-Brücke bilden, die bis zu 3 C₁- bis C₄-Alkylreste tragen kann, und
n für eine ganze Zahl von 1 bis 3 steht.

2. UV-geschützte elektrochrome Lösung gemäß Anspruch 1, **dadurch gekennzeichnet, dass** sie einen UV-Absorber der Formel 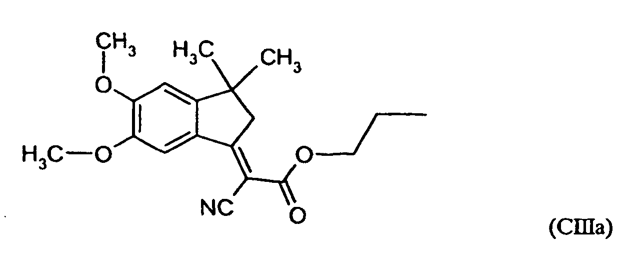 erhält.

3. UV-geschützte elektrochrome Lösung gemäß Anspruch 1 oder 2, **dadurch gekennzeichnet, dass** sie den UV-Absorber in Konzentrationen von 0,01 bis 2 mol/l, enthält.

4. Verwendung der elektrochromen Lösung gemäß Ansprüchen 1 bis 3 als Medium mit variabler Transmission in einer elektrochromen Vorrichtung.

5. Elektrochrome Vorrichtung enthaltend eine elektrochrome Lösung gemäß Ansprüchen 1 bis 3.

6. Elektrochrome Vorrichtung gemäß Anspruch 5, **dadurch gekennzeichnet, dass** sie als Zelle, als Fensterscheibe, Spiegel, Sonnendach oder Anzeigevorrichtung aus strukturierten, einzeln kontaktierbaren Segmenten ausgebildet ist.

7. Elektrochrome Vorrichtung gemäß Ansprüchen 5 oder 6, **dadurch gekennzeichnet, dass** sie aus zwei einander zugewandten lichtdurchlässigen Glas- oder Kunststoffscheiben besteht, von denen gegebenenfalls eine verspiegelt ist und deren einander zugewandten Seiten elektrisch leitfähig beschichtet und gegebenenfalls in Segmente aufgeteilt sind zwischen denen die elektrochrome Flüssigkeit enthalten ist.

## Claims

1. UV-protected electrochromic solution comprising at least one oxidizable substance RED₁ which releases electrons at an anode, and at least one reducible substance OX₂ which accepts electrons at a cathode and in so doing undergo transition from a weakly coloured or colourless form into a coloured form OX₁ and RED₂, respectively, accompanied by an increase in the absorbance in the visible region of the spectrum, the weakly coloured or colourless form being restored after charge equalization, **characterized in that**
it comprises at least one electrochromic substance of the formula
OX₂-B-RED₁ (Ia),
OX₂-B-RED₁-B-OX₂ (Ib),
RED₁-B-OX₂-B-RED₁ (Ic),
or
OX₂-(B-RED₁-B-OX₂)_{d}-B-RED₁ (Id),
in which
d represents an integer from 1 to 5.
OX₂ represents a radical of the formulae 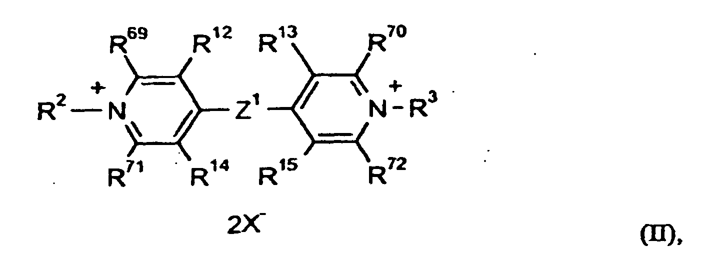 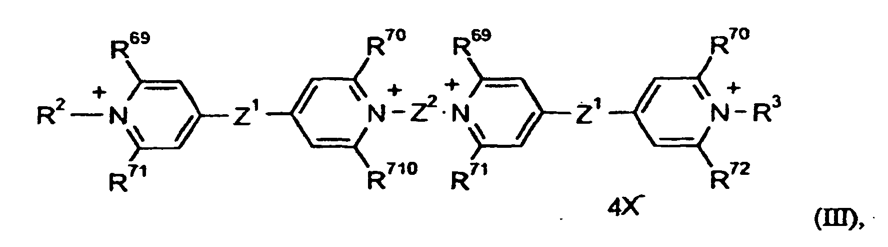 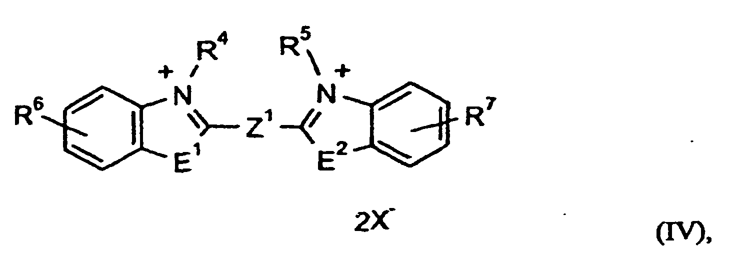 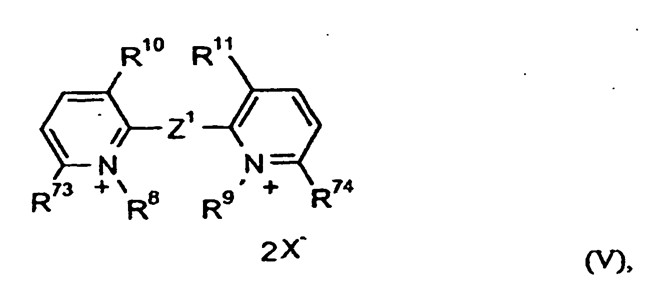 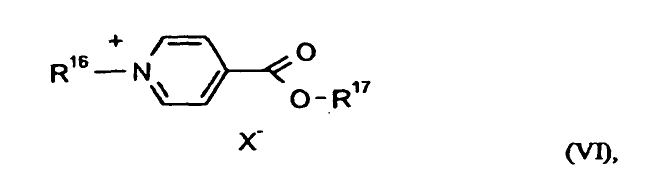 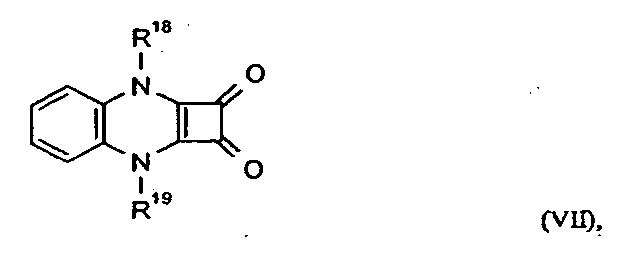 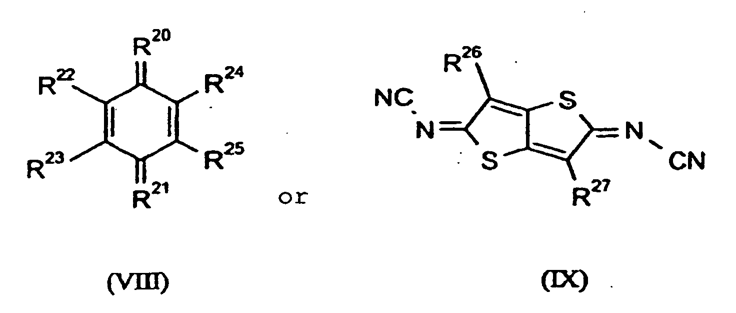
where
R² to R⁵, R⁸, R⁹, R¹⁶ to R¹⁹ independently of one another denote C₁- to C₁₈-alkyl, C₂- to C₁₂-alkenyl, C₃- to C₇-cycloalkyl, C₇- to C₁₅-aralkyl or C₆- to C₁₀-aryl, or
R⁴ and R⁵ or R⁸ and R⁹ together form a -(CH₂)₂- or -(CH₂)₃- bridge,
R⁶, R⁷and R²² to R²⁵ independently of one another denote hydrogen, C₁- to C₄-alkyl, C₁- to C₄-alkoxy, halogen, cyano, nitro or C₁- to C₄-alkoxycarbonyl, or
R²² and R²³ and/or R²⁴ and R²⁵ form a -CH=CH-CH=CH- bridge,
R¹⁰ and R¹¹; R¹² and R¹³; R¹⁴ and R¹⁵ independently of one another denote hydrogen or in pairs denote a -(CH₂)₂-, -(CH₂)₃- or -CH=CH-bridge,
R⁶⁹ toR⁷⁴ independently of one another denote hydrogen or C₁-C₆-alkyl, or
R⁶⁹; R¹² and/or R⁷⁰; R¹³ form a -CH=CH-CH=CH- bridge,
R²⁰ and R²¹ independently of one another denote O, N-CN, C(CN)₂ or N-C₆-to C ₁₀-aryl,
R²⁶ denotes hydrogen, C₁- to C₄-alkyl, C₁- to C₄-alkoxy, halogen, cyano, nitro, C₁- to C₄-alkoxycarbonyl or C₆- to C₁₀-aryl,
E¹ and E² independently of one another denote O, S, NR¹ or C(CH₃)₂, or
E¹ and E² together form an -N-(CH₂)₂-N- bridge,
R¹ denotes C₁- to C₁₈-alkyl, C₂- to C₁₂-alkenyl, C₄- to C₇-cycloalkyl, C₇-to C₁₅-aralkyl, C₆- to C₁₀-aryl,
Z¹ denotes a direct bond, -CH=CH-, -C(CH₃)=CH-, -C(CN)=CH-, -CCl=CCl-, -C(OH)=CH-, -CCl=CH-, -C=C-, -CH=N-N=CH-, -C(CH₃)=N-N=C(CH₃)- or -CCl=N-N=CCl-,
Z² denotes -(CH₂)ᵣ- or -CH₂-C₆H₄-CH₂-,
r denotes an integer from 1 to 10, and
X⁻ denotes an anion which is redox-inert under the conditions
where the bond to the bridge B is via one of the radicals R²-R¹⁹, R²²-R²⁷ or, if E¹ or E² represents NR¹, is via R¹, and the radicals mentioned **in that** case represent a direct bond,
RED₁ represents one of the following radicals 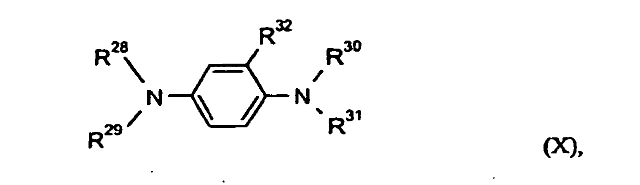 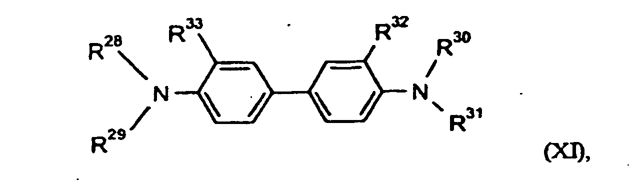 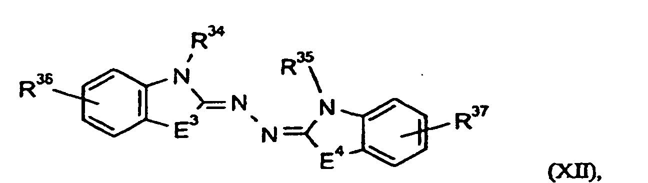 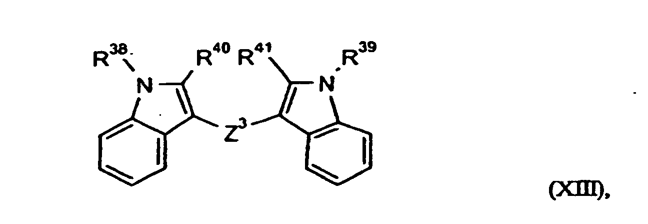 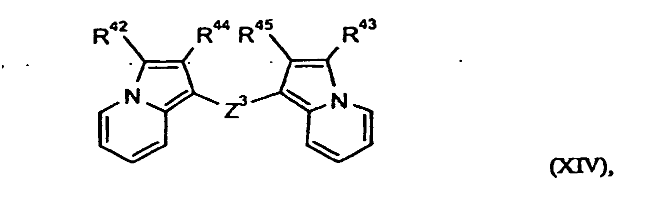 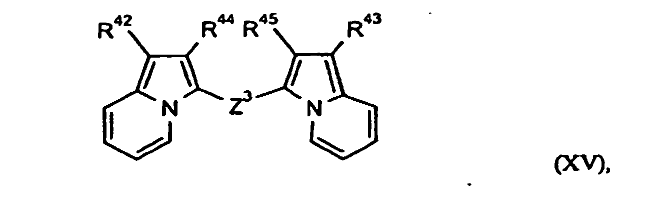 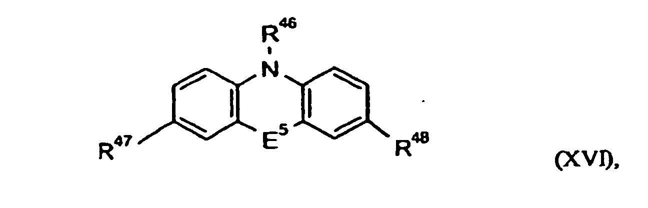 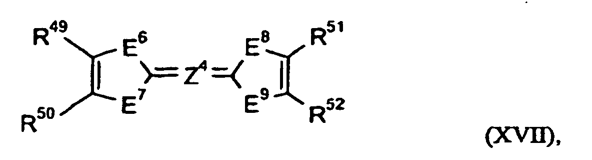 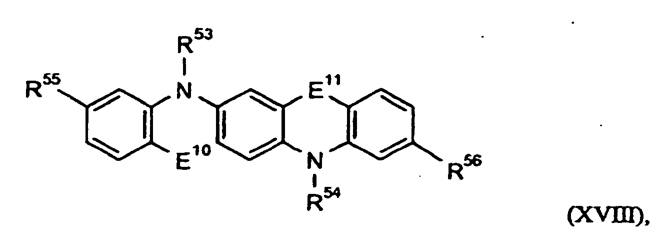 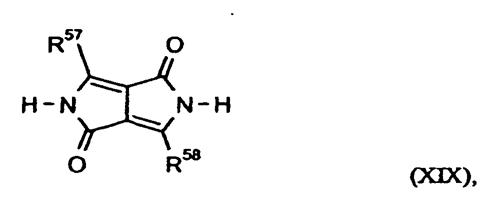 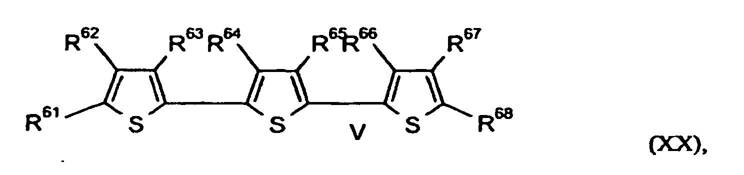
in which
R²⁸ to R³¹, R³⁴, R³⁵, R³⁸, R³⁹, R⁴⁶, R⁵³ and R⁵⁴ independently of one another denote C₁- to C₁₈-alkyl, C₂- to C₁₂-alkenyl, C₃- to C₇-cycloalkyl, C₇- to C₁₅-aralkyl or C₆- to C₁₀-aryl, and R⁴⁶, R⁵³ and R⁵⁴ additionally denote hydrogen,
R³², R³³, R³⁶, R³⁷, R⁴⁰, R⁴¹, R⁴² to R⁴⁵, R⁴⁷, R⁴⁸, R⁴⁹ to R⁵² and R⁵⁵ to R⁵⁷ independently of one another denote hydrogen, C₁- to C₄-alkyl, C₁- to C₄-alkoxy, halogen, cyano, nitro, C₁- to C₄-alkoxycarbonyl or C₆- to C₁₀-aryl and R⁵⁷ and R⁵⁸ additionally denote an optionally benzofused aromatic or quasiaromatic five- or six-membered heterocyclic ring and R⁴⁸ additionally denotes NR⁷⁵R⁷⁶,
R⁴⁹ and R⁵⁰ and/or R⁵¹ and R⁵² form a -(CH₂)₃-, -(CH₂)₄-, -(CH₂)₅- or -CH=CH-CH=CH- bridge,
Z³ denotes a direct bond, a -CH=CH- or -N=N- bridge,
=Z⁴= denotes a direct double bond, a =CH-CH= or =N-N= bridge,
E³ to E⁵, E¹⁰and E¹¹ independently of one another denote O, S, NR⁵⁹ or C(CH₃)₂, and E⁵ additionally denotes C=O or SO₂, or
E¹³ and E⁴ independently of one another denote -CH=CH-,
E⁶ to E⁹ independently of one another denote S, Se or NR⁵⁹,
R⁵⁹, R⁷⁵ and R⁷⁶ independently of one another denote C₁- to C₁₂-alkyl, C₂- to C₈-alkenyl, C₃- to C₇-cycloalkyl, C₇- to C₁₅-aralkyl or C₆- to C₁₀-aryl, and R⁷⁵ additionally denotes hydrogen, or
R⁷⁵ and R⁷⁶ in the definition of NR⁷⁵R⁷⁶ form, together with the N atom to which they are attached, a five- or six-membered, saturated ring which can contain further heteroatoms,
R⁶¹ to R⁶⁸ independently of one another denote hydrogen, C₁- to C₆-alkyl, C₁- to C₄-alkoxy, cyano, C₁- to C₄-alkoxycarbonyl or C₆- to C₁₀aryl, or
R⁶¹; R⁶²and R⁶⁷; R⁶⁸ independently of one another, together form a -(CH₂)₃-, -(CH₂)₄- or -CH=CH-CH=CH- bridge,
v denotes an integer between 0 and 10,
the bond to the bridge B being via one of the radicals R²⁸-R⁵⁸, R⁶¹, R⁶², R⁶⁷, R⁶⁸ or, if one of the radicals E³-E¹¹ represents NR⁵⁹, is via R⁵⁹ and the abovementioned radicals **in that** case represent a direct bond, and
B represents a bridge of the formula -(CH₂)ₙ- or -[Y¹ₛ(CH₂)ₘ-Y²]ₒ-(CH₂)ₚ-Y³_{q}-, each of which is unsubstituted or substituted by C₁- to C₄-alkoxy, halogen or phenyl,
Y¹ to Y³ independently of one another represent O, S, NR⁶⁰, COO, CONH, NHCONH, cyclopentanediyl, cyclohexanediyl, phenylene or naphthylene,
R⁶⁰ denotes C₁- to C₆-alkyl, C₂- to C₆-alkenyl, C₄- to C₇-cycloalkyl, C₇- to C₁₅-aralkyl or C₆- to C₁₀-aryl,
n denotes an integer from 1 to 12,
m and p independently of one another denote an integer from 0 to 8,
o denotes an integer from 0 to 6, and
q and s independently of one another denote 0 or 1,
and comprises a UV absorber of the formula 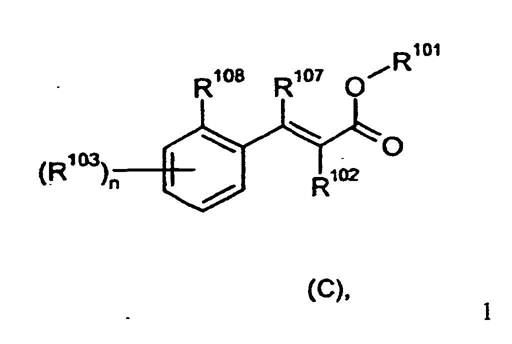 in which
R¹⁰¹ represents linear or branched C₁- to C₂₀-alkyl,
R¹⁰² represents hydrogen, cyano or COOR¹,
R¹⁰³ represents hydrogen, C₁- to C₁₂-alkyl or C₁- to C₁₂-alkoxy, and
R¹⁰⁷ together with R¹⁰⁸ forms a C₂ or C₃ bridge which can carry up to 3 C₁- to C₄-alkyl radicals, and
n represents an integer from 1 to 3.

2. UV-protected electrochromic solution according to Claim 1, **characterized in that** it comprises a UV absorber of the formula

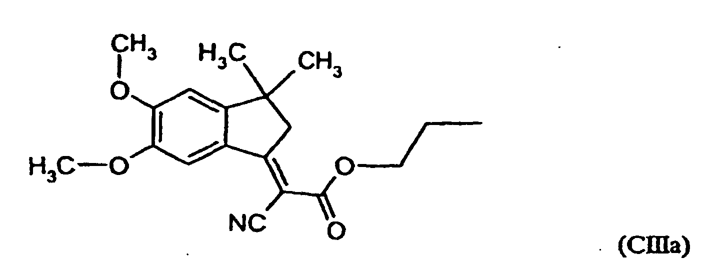

3. UV-protected electrochromic solution according to Claim 1 or 2, **characterized in that** it comprises the UV absorbers in concentrations of from 0.01 to 2 mol/l.

4. Use of the electrochromic solution according to Claims 1 to 3 as a medium with variable transmission in an electrochromic device.

5. Electrochromic device comprising an electrochromic solution according to Claims 1 to 3.

6. Electrochromic device according to Claim 5, **characterized in that** it is configured as a cell, as a window pane, mirror, sunroof or display device and comprises structured, individually contactable segments.

7. Electrochromic device according to Claims 5 and 6, **characterized in that** it consists of two facing, transparent glass or plastic plates of which one may be mirrored and whose facing sides carry an electroconductive coating and may be subdivided into segments between which the electrochromic fluid is contained.

## Revendications

1. Solution électrochrome protégée contre les UV, contenant au moins une substance oxydable RED₁, qui, par perte d'électrons au niveau d'une anode, et au moins une substance réductible OX₂, qui, par gain d'électrons au niveau d'une cathode, avec augmentation de l'extinction dans le domaine visible du spectre, passe d'une forme faiblement colorée
ou incolore à une forme colorée OX₁ ou RED₂, où, après égalisation des charges, la forme faiblement colorée ou incolore est formée de nouveau,
**caractérisée**
**en ce qu'**elle contient au moins une substance électrochrome des formules
OX₂-B-RED₁ (Ia),
OX₂-B-RED₁-B-OX₂ Ib),
RED₁-B-OX₂-B-RED₁ (Ic),
ou
OX₂-(B-RED₁-B-OX₂)_{d}-B-RED₁ (Id),
où
d représente un nombre entier de 1 à 5,
OX₂ représente un groupement des formules 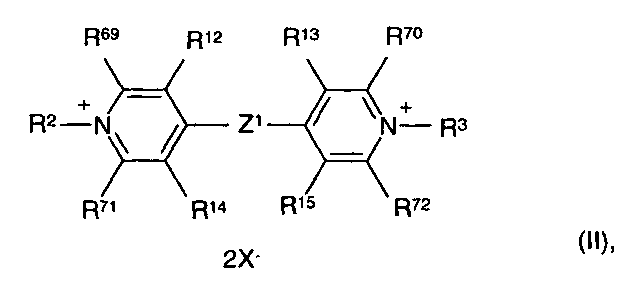 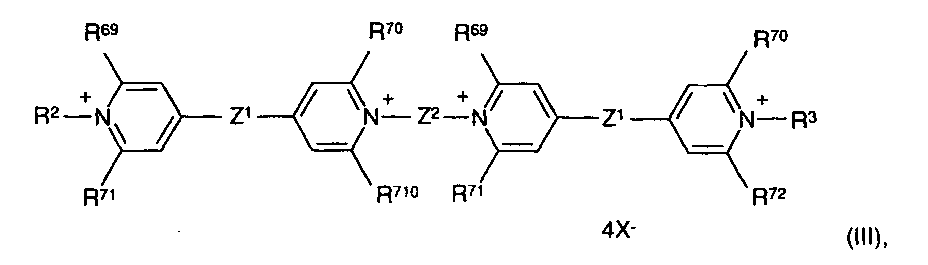 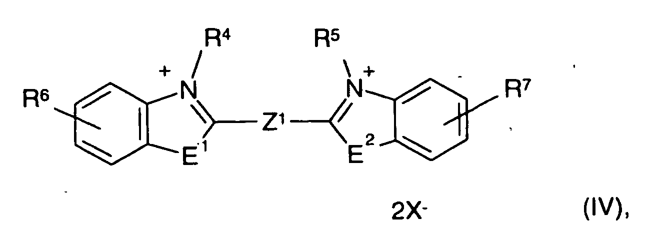 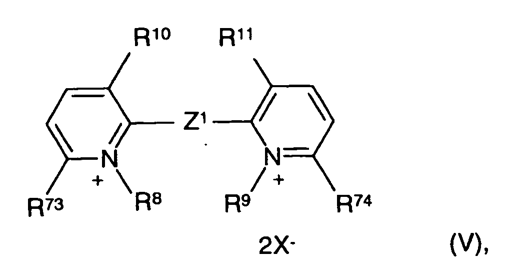 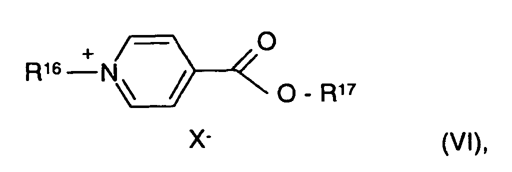 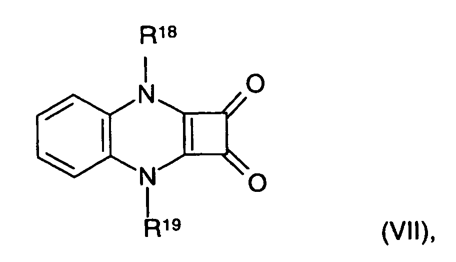 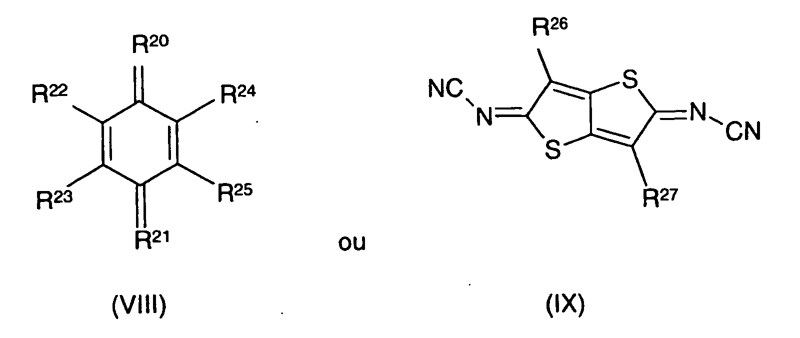
où
R² à R⁵, R⁸, R⁹, R¹⁶ à R¹⁹ représentent indépendamment les uns des autres C₁- à C₁₈-alkyle, C₂- à C₁₂-alcényle, C₃- à C₇-cycloalkyle, C₇- à C₁₅-aralkyle ou C₆- à C₁₀-aryle
ou
R⁴ et R⁵ ou R⁸ et R⁹ forment ensemble un pont -(CH₂)₂- ou -(CH₂)₃-,
R⁶, R⁷ et R²² à R²⁵ représentent indépendamment les uns des autres l'hydrogène, C₁- à C₄-alkyle, C₁- à C₄-alcoxy, halogène, cyano, nitro ou C₁- à C₄-alcoxycarbonyle ou
R²² et R²³ et/ou R²⁴ et R²⁵ forment un pont -CH=CH-CH=CH-,
R¹⁰ et R¹¹ ; R¹² et R¹³ ; R¹⁴ et R¹⁵ représentent indépendamment les uns des autres l'hydrogène ou par paires un pont -(CH₂)₂-, -(CH₂)₃- ou -CH=CH-,
R⁶⁹ à R⁷⁴ représentent indépendamment les uns des autres l'hydrogène ou C₁-C₆-alkyle ou
R⁶⁹ ; R¹² et/ou R⁷⁰ ; R¹³ forment un pont -CH=CH-CH=CH-,
R²⁰ et R²¹ représentent indépendamment l'un de l'autre O, N-CN, C(CN)₂ ou N-C₆- à C₁₀-aryle,
R²⁶ représente l'hydrogène, C₁- à C₄-alkyle, C₁- à C₄-alcoxy, halogène, cyano, nitro, C₁- à C₄-alcoxycarbonyle ou C₆- à C₁₀-aryle,
E¹ et E² représentent indépendamment l'un de l'autre O, S, NR¹ ou C(CH₃)₂ ou
E¹ et E² forment ensemble un pont -N-(CH₂)₂-N-,
R¹ représente C₁- à C₁₈-alkyle, C₂- à C₁₂-alcényle, C₄- à C₇-cycloalkyle, C₇- à C₁₅-aralkyle, C₆- à C₁₀-aryle,
Z¹ représente une liaison directe, -CH=CH-, -C(CH₃)=CH-, -C(CN)=CH-, -CCl=CCl-, -C(OH)=CH-, -CCI=CH-, -C=C-, -CH=N-N=CH-, -C(CH₃)=N-N=C(CH₃)- ou -CCl=N-N=CCl-,
Z² représente -(CH₂)ᵣ ou -CH₂-C₆H₄-CH₂-,
r représente un nombre entier de 1 à 10 et
X⁻ représente un anion inerte du point de vue redox dans les conditions,
où la liaison au membre de pont B a lieu par le biais de l'un des groupements R²-R¹⁹, R²²-R²⁷ ou dans le cas où E¹ ou E² représente NR¹ par le biais de R¹ et les groupements cités représentent alors une liaison directe,
RED₁ représente l'un des groupements suivants 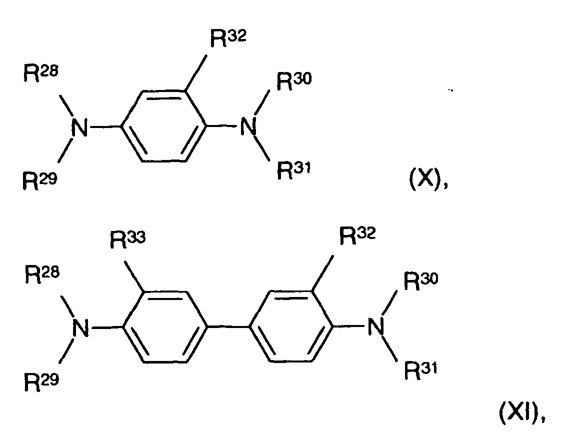 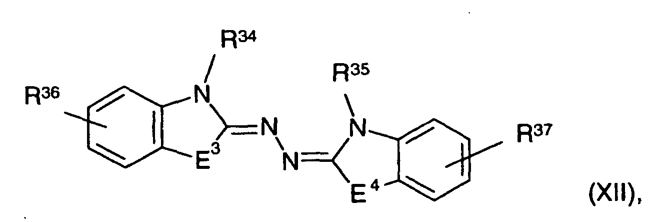 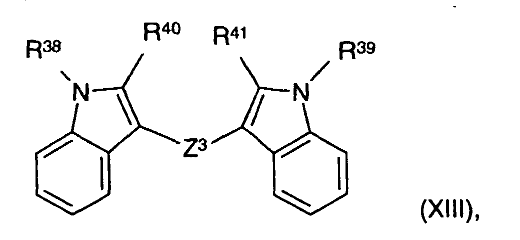 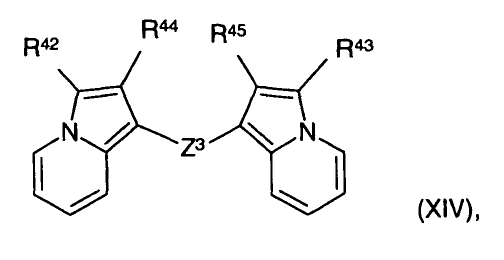 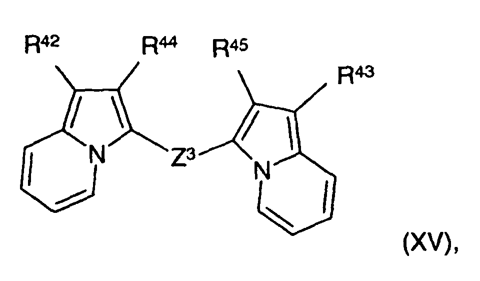 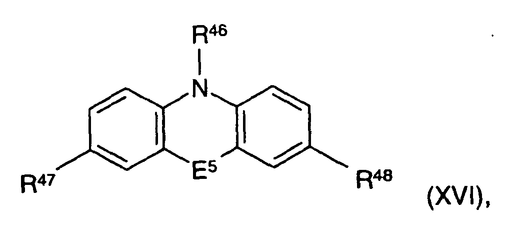 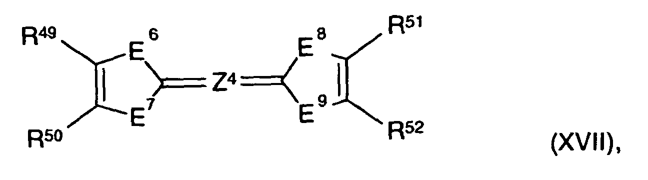 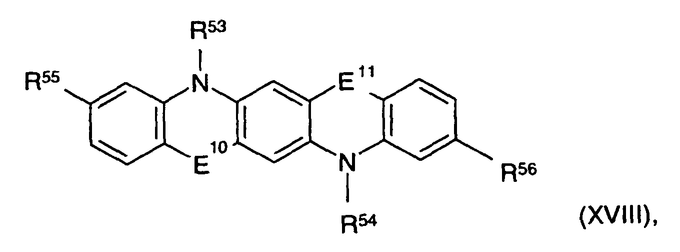 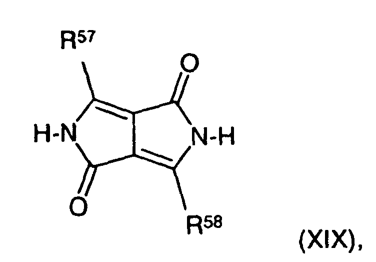 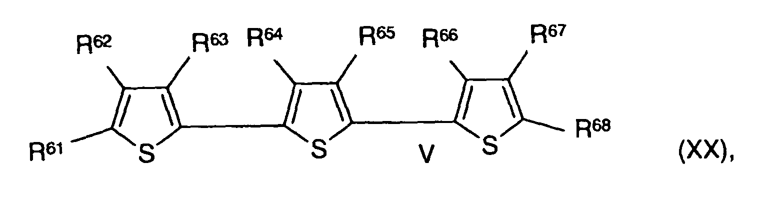
où
R²⁸ à R³¹, R³⁴, R³⁵, R³⁸, R³⁹, R⁴⁶, R⁵³ et R⁵⁴ représentent indépendamment les uns des autres C₁- à C₁₈-alkyle, C₂- à C₁₂-alcényle, C₃- à C₇-cycloalkyle, C₇- à C₁₅-aralkyle ou C₆- à C₁₀-aryle et R⁴⁶, R⁵³ et R⁵⁴ représentent en outre l'hydrogène,
R³², R³³, R³⁶, R³⁷, R⁴⁰, R⁴¹, R⁴² à R⁴⁵, R⁴⁷, R⁴⁸, R⁴⁹ à R⁵² et R⁵⁵ à R⁵⁷ représentent indépendamment les uns des autres l'hydrogène, C₁- à C₄-alkyle, C₁- à C₄-alcoxy, halogène, cyano, nitro, C₁- à C₄-alcoxycarbonyle ou C₆- à C₁₀-aryle et R⁵⁷ et R⁵⁸ représentent en outre un cycle hétérocyclique à 5 ou 6 chaînons aromatique ou quasi-aromatique éventuellement benzocondensé et R⁴⁸ représente en outre NR⁷⁵R⁷⁶,
R⁴⁹ et R⁵⁰ et/ou R⁵¹ et R⁵² forment un pont -(CH₂)₃-, -(CH₂)₄-, -(CH₂)₅- ou -CH=CH-CH=CH-,
Z³ représente une liaison directe, un pont -CH=CH- ou -N=N-,
=Z⁴= représente une double liaison directe, un pont =CH-CH= ou =N-N=,
E³ à E⁵, E¹⁰ et E¹¹ représentent indépendamment les uns des autres O, S, NR⁵⁹ ou C(CH₃)₂ et E⁵ représente en outre C=O ou SO₂, ou
E³ et E⁴ représentent indépendamment l'un de l'autre -CH=CH-,
E⁶ à E⁹ représentent indépendamment les uns des autres S, Se ou NR⁵⁹,
R⁵⁹, R⁷⁵ et R⁷⁶ représentent indépendamment les uns des autres C₁- à C₁₂-alkyle, C₂- à C₈-alcényle, C₃- à C₇-cycloalkyle, C₇- à C₁₅-aralkyle ou C₆- à C₁₀-aryle et R⁷⁵ représente en outre l'hydrogène ou
R⁷⁵ et R⁷⁶ dans la signification de NR⁷⁵R⁷⁶ forment avec l'atome N auquel ils sont liés un cycle saturé à 5 ou 6 chaînons qui peut contenir d'autres hétéroatomes,
R⁶¹ à R⁶⁸ représentent indépendamment les uns des autres l'hydrogène, C₁- à C₆-alkyle, C₁- à C₄-alcoxy, cyano, C₁- à C₄-alcoxycarbonyle ou C₆- à C₁₀-aryle, ou
R⁶¹ ; R⁶² et R⁶⁷ ; R⁶⁸ forment ensemble indépendamment les uns des autres un pont -(CH₂)₃-, -(CH₂)₄- ou -CH=CH-CH=CH-,
v représente un nombre entier entre 0 et 10,
où la liaison avec le membre de pont B a lieu par le biais de l'un des groupements R²⁸-R⁵⁸, R⁶¹, R⁶², R⁶⁷, R⁶⁸ ou bien, dans le cas où l'un des groupements E³-E¹¹ représente NR⁵⁹, par le biais de R⁵⁹ et les groupements cités représentent alors une liaison directe, et
B représente un membre de pont des formules -(CH₂)ₙ ou -[Y¹ₛ(CH₂)ₘ-Y²]ₒ-(CH₂)ₚ-Y³_{q}- qui sont éventuellement substitués dans chaque cas par C₁- à C₄-alcoxy, halogène ou phényle,
Y¹ à Y³ représentent indépendamment l'un de l'autre O, S, NR⁶⁰, COO, CONH, NHCONH, cyclopentanediyle, cyclohexanediyle, phénylène ou naphtylène,
R⁶⁰ représente C₁- à C₆-alkyle, C₂- à C₆-alcényle, C₄- à C₇-cycloalkyle, C₇- à C₁₅-aralkyle ou C₆- à C₁₀-aryle,
n représente un nombre entier de 1 à 12,
m et p représentent indépendamment l'un de l'autre un nombre entier de 0 à 8,
o représente un nombre entier de 0 à 6 et
q et s représentent indépendamment l'un de l'autre 0 ou 1,
et un absorbeur d'UV de formule 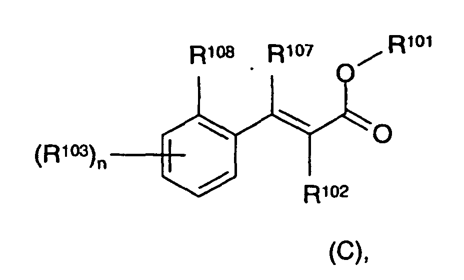 où
R¹⁰¹ représente C₁- à C₂₀-alkyle éventuellement ramifié,
R¹⁰² représente l'hydrogène, cyano ou COOR¹,
R¹⁰³ représente l'hydrogène, C₁- à C₁₂-alkyle ou C₁- à C₁₂-alcoxy,
R¹⁰⁷ forme avec R¹⁰⁸ un C₂- ou C₃-pont, qui peut porter jusqu'à 3 groupements C₁- à C₄-alkyle, et
n représente un nombre entier de 1 à 3.

2. Solution électrochrome protégée contre les UV selon la revendication 1, **caractérisée en ce qu'**elle contient un absorbeur d'UV de formule

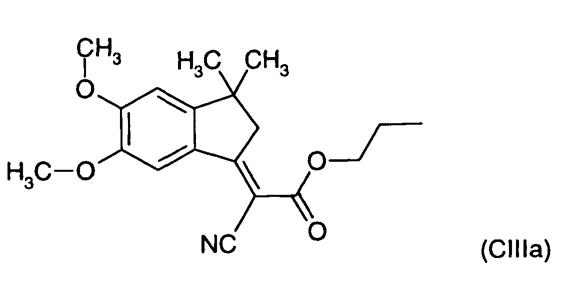

3. Solution électrochrome protégée contre les UV selon la revendication 1 ou 2, **caractérisée en ce qu'**elle contient l'absorbeur d'UV en des concentrations de 0,01 à 2 mol/l.

4. Utilisation de la solution électrochrome selon les revendications 1 à 3 comme milieu à transmission variable dans un dispositif électrochrome.

5. Dispositif électrochrome contenant une solution électrochrome selon les revendications 1 à 3.

6. Dispositif électrochrome selon la revendication 5, **caractérisé en ce qu'**il est sous forme de cellule, de vitre, de miroir, de toit ouvrant ou de dispositif indicateur en segments structurés pouvant être mis en contact individuellement.

7. Dispositif électrochrome selon les revendications 5 ou 6, **caractérisé en ce qu'**il consiste en deux plaques de verre ou de matière plastique transparentes tournées l'une vers l'autre parmi lesquelles l'une est éventuellement métallisée et dont les côtés tournés l'un vers l'autre sont munis d'un revêtement électriquement conducteur et sont éventuellement divisés en segments entre lesquels est contenu le liquide électrochrome.
